# EUROPEAN PATENT APPLICATION

(11) **EP 3 369 821 A1**
(43) Date of publication of application: **05.09.2018**
(21) Application number: 16857835.9
(22) Date of filing: 21.10.2016
(51) Int. Cl.: C12N 15/77, C12N 9/10, C12P 13/06

(54) **CORYNEBACTERIUM SP. MICROORGANISM HAVING L-ISOLEUCINE PRODUCING ABILITY AND METHOD FOR PRODUCING L-ISOLEUCINE BY USING SAME**

(30) Priority: 23.10.2015 KR 20150148157
(71) Applicant: Cj Cheiljedang Corporation, Seoul 04560 (KR)
(72) Inventor: KWON, Su Yon, Suwon-si Gyeonggi-do 16509 (KR); JANG, Jae Woo, Hwaseong-si Gyeonggi-do 18444 (KR); KIM, Min Se, Seoul 07538 (KR); KIM, Ju Jeong, Suwon-si Gyeonggi-do 16225 (KR)
(74) Representative: Schiweck Weinzierl Koch Patentanwälte Partnerschaft mbB
(86) International application number: PCT/KR2016/011923
(87) International publication number: WO 2017/069578

(57) **Abstract**

The present application relates to a microorganism of the genus *Corynebacterium* having L-isoleucine producing ability which comprises a protein having an activity of citramalate synthase , and a method for producing L-isoleucine using the same.

## Description

### [Technical Field]

The present disclosure relates to a microorganism of the genus *Corynebacterium* having L-isoleucine producing ability, and a method for producing L-isoleucine using the same.

### [Background Art]

Branched-chain amino acids refer to the three amino acids L-valine, L-leucine, and L-isoleucine, and are industrially used as food additives, pharmaceutical agents, *etc.* In particular, L-isoleucine is metabolized in muscles to generate energy, and is involved in the generation of hemoglobin while functioning to relieve fatigue and promote growth. Accordingly, L-isoleucine has various uses as a fluid preparation, nutritional supplement, *etc.,* and its use as a workout supplement is increasing.

L-isoleucine is biosynthesized from the precursors pyruvate and 2-ketobutyrate through three metabolic intermediates (Jinhwan Park et al., Appl. Microbial. Biotechnol. 85: 491-506, 2010).

However, in the biosynthesis of L-isoleucine, threonine dehydratase (ilvA gene), which produce 2-ketobutyrate from L-threonine, and then acetohydroxy acid synthase (ilvBN gene) are both subject to feedback inhibition by L-isoleucine.

Accordingly, it is known that regulation of enzymes involved in biosynthesis by release from feedback inhibition by L-isoleucine is an important element in preparing a strain producing a high yield of L-isoleucine (Jinhwan Park et al., Biotechnology Journal, 560-577, 2010). Further, branched-chain amino acids are generated through the same biosynthesis pathways as far as pyruvate, and accordingly, mass production of L-isoleucine at high yield requires smooth provision of L-threonine in the step prior to 2-ketobutyrate (Korean Patent No. 10-0823044).

### [Disclosure]

### [Technical Problem]

The present inventors examined a novel biosynthesis pathway which does not use L-threonine as a precursor, and introduced a gene having an activity of citramalate synthase into the biosynthesis pathway of L-isoleucine. As a result, the inventors confirmed that the introduction improves the production of L-isoleucine, thereby completing the present disclosure.

### [Technical Solution]

An objective of the present disclosure is to provide a recombinant microorganism having an introduced novel biosynthesis pathway and having L-isoleucine producing ability.

Another objective of the present disclosure is to provide a method for producing L-isoleucine, comprising culturing there recombinant microorganism having an introduced novel biosynthesis pathway in a medium; and recovering L-isoleucine from the microorganism or medium.

### [Advantageous Effect]

The microorganism of the genus *Corynebacterium* having L-isoleucine producing ability of the present disclosure has an introduced activity of citramalate synthase, and can produce a high yield of L-isoleucine through a novel biosynthesis pathway where L-threonine is not used as a precursor.

### [Preferred Embodiment of Invention]

An aspect of the present disclosure to achieve the objects provides a microorganism of the genus *Corynebacterium* having L-isoleucine producing ability and comprising a protein having an activity of citramalate synthase.

The term "L-isoleucine" in the present disclosure refers to an essential amino acid, and structurally, to the L-amino acid of the formula HO₂CCH(NH₂)CH(CH₃)CH₂CH₃, corresponding to a branched-chain amino acid together with L-valine and L-leucine.

The phrase "having L-isoleucine producing ability" in the present disclosure indicates that when a related microorganism is cultured in a medium, it exhibits an ability to accumulate L-isoleucine in the medium or the microorganism itself. Such L-isoleucine producing ability may be inherited by a wild-type strain or provided with or enhanced by modifying a strain.

For example, for having L-isoleucine producing ability, the microorganism may adopt methods used for modification of a microorganism solely or in combination, such as acquisition of an auxotrophic, analogue resistant, or metabolism control mutant, or construction of a recombinant strain in which an activity of an enzyme involved in the L-isoleucine biosynthesis is enhanced.

Further, in the case of enhancing an activity of an enzyme involved in the L-isoleucine biosynthesis, the targeted gene involved in the L-isoleucine biosynthesis, specifically ilvG, ilvM, ilvE, ilvD, and ilvA genes, may be enhanced solely or in a combination of two or more genes. The ilvG, ilvM, ilvE, ilvD, and ilvA genes encode large and small subunits of isozyme II of acetohydroxy acid synthase, transaminase, dihydroxy acid dehydratase, and threonine deaminase, respectively.

The term "citramalate synthase (EC2.3.1.182)" in the present disclosure is a catalytic enzyme for transforming acetyl-CoA and pyruvate to citramalate and coenzyme A. This enzyme is found in Archaea such as *Methanocaldococcus jannaschii* (Howell et al., J Bacteriol. 181: 331-333, 1999), *Leptospira interogans* (Xu et al., J Bacteriol. 186: 5400-5409, 2004), and *Geobacter sulfurreducens,* and is involved in a threonine-independent pathway in the biosynthesis of isoleucine (Risso et al., J Bacteriol. 190: 2266-2274, 2008). The enzyme has a high specificity to pyruvate as a substrate, and is known as typically being inhibited by isoleucine.

The terms "a protein having an activity of citramalate synthase" and "a gene encoding the same" in the present disclosure may include any proteins having an activity of citramalate synthase as described above and any genes encoding the same without limitation.

Specifically, a protein having an activity of citramalate synthase may be derived from *Methanocaldococcus,* and more specifically, *Methanocaldococcus jannaschii.* The citramalate synthase has the amino acid sequence of SEQ ID NO: 1, and cimA gene encoding the same has a nucleotide sequence of SEQ ID NO: 2. Amino acid sequences of a protein showing the activity are different depending on the species or strain of microorganism, or multiple nucleic acids having an identical function may encode any predetermined proteins because of degeneracy of a genetic code, and accordingly, the sequences are not limited to the SEQ ID NOS described above.

Further, any proteins substantially having an activity of citramalate synthase may be included in the scope of the present disclosure without limitation as long as the protein substantially has an activity of citramalate synthase as an amino acid sequence showing 70% or more homology to SEQ ID NO: 1, specifically 80% or more homology thereto, more specifically 90% or more homology thereto, more specifically 95% or more homology thereto, and even more specifically 98% or more homology thereto.

Additionally, if an amino acid sequence with the homology to the sequences substantially has a biological activity identical or corresponding to a protein of SEQ ID NO: 1, an amino acid sequence whose portion is deleted, modified, substituted, or added is also obviously included in the scope of the present disclosure.

Further, a gene encoding the citramalate synthase may have a nucleotide sequence encoding the amino acid sequence of SEQ ID NO: 1. Herein, because of the degeneracy of a codon or considering a codon preferred by an organism to express the protein, a polynucleotide may have various transformations in the encoding regions within the scope where an amino acid sequence of the protein is not changed. The polynucleotide sequence may, for example, have the polynucleotide sequence of SEQ ID NO: 2, and a nucleotide sequence having 80% or more homology thereto, specifically 90% or more homology thereto, and more specifically 99% or more homology thereto, but is not limited thereto.

Further, in order to recover the mutant having an enhanced activity of citramalate synthase, the present disclosure provides a mutant library of CimA(M) recovered using error-prone PCR as a mutant of a protein of SEQ ID NO: 1.

Specifically, the mutant library of CimA(M) of the present disclosure may include the following:
CimA(M)m1 having the amino acid sequence of SEQ ID NO: 3 (specifically, possibly being encoded from a polynucleotide having a nucleotide sequence of SEQ ID NO: 4);
CimA(M)m2 having the amino acid sequence of SEQ ID NO: 5 (specifically, possibly being encoded from a polynucleotide having a nucleotide sequence of SEQ ID NO: 6);
CimA(M)m3 having the amino acid sequence of SEQ ID NO: 7 (specifically, possibly being encoded from a polynucleotide having a nucleotide sequence of SEQ ID NO: 8);
CimA(M)m4 having the amino acid sequence of SEQ ID NO: 9 (specifically, possibly being encoded from a polynucleotide having a nucleotide sequence of SEQ ID NO: 10);
CimA(M)m5 having the amino acid sequence of SEQ ID NO: 11 (specifically, possibly being encoded from a polynucleotide having a nucleotide sequence of SEQ ID NO: 12);
CimA(M)m6 having the amino acid sequence of SEQ ID NO: 13 (specifically, possibly being encoded from a polynucleotide having a nucleotide sequence of SEQ ID NO: 14); and
CimA(M)m7 having the amino acid sequence of SEQ ID NO: 15 (specifically, possibly being encoded from a polynucleotide having a nucleotide sequence of SEQ ID NO: 16).

Further, any proteins substantially having an activity of a citramalate synthase mutant may be included in the scope of the present disclosure without limitation as long as the protein substantially has an activity of the citramalate synthase mutant as an amino acid sequence showing a homology to amino acid sequences of the SEQ ID NOS of specifically 80% or more, more specifically 90% or more, even more specifically 95% or more, and far even more specifically 99% or more.

The term "homology" in the present disclosure refers to a degree of similarity to a given amino acid or nucleotide sequence, and may be represented as a percentage. In the present disclosure, homology of a sequence having an activity identical or similar to a given amino acid or nucleotide sequence is represented as "% homology". For example, homology may be identified using standard software, specifically BLAST 2.0, for calculating parameters such as score, identity, and similarity or by comparing sequences through Southern hybridization under defined stringent conditions. Appropriate hybridization conditions may be defined within the scope of the art (*e.g.,* J. Sambrook et al., Molecular Cloning, A Laboratory Manual, 1989), and may be determined using methods well known by one of ordinary skill in the art.

In a specific embodiment, a microorganism of the present disclosure may include citramalate synthase of SEQ ID NO: 1 derived from *Methanocaldococcus jannaschii,* a mutant thereof, and a gene encoding a protein of an amino acid sequence selected from the group consisting of SEQ ID NOS: 1, 3, 5, 7, 9, 11, 13, and 15. More specifically, a desired gene may be expressed by transforming recombinant vectors including each gene into the microorganism.

The term "recombinant vector" in the present disclosure refers to a DNA construct containing a nucleotide sequence of a polynucleotide encoding a desired protein, which is operably linked to an appropriate regulatory sequence to express the desired protein in a suitable host. The regulatory sequence includes a promoter that can initiate transcription, an optional operator sequence for regulating such transcription, a sequence encoding a suitable mRNA ribosome binding site, and a sequence regulating the termination of transcription and translation. After the recombinant vector is transformed into a suitable host cell, it can replicate or function regardless of a genome of the host, and can be integrated into the genome itself.

A recombinant vector used in the present disclosure is not particularly limited as long as it is able to replicate in a host cell, and any vectors known in the art may be used. Examples of conventionally used vectors may include a natural or recombinant plasmid, cosmid, virus, and bacteriophage. For example, as a phage vector or cosmid vector, pWE15, M13, MBL3, MBL4, IXII, ASHII, APII, t10, t11, Charon4A, Charon21A, or the like may be used. As a plasmid vector, a pBR type, pUC type, pBluescriptII type, pGEM type, pTZ type, pCL type, pET type, or the like may be used. Vectors usable in the present disclosure are not particularly limited, and any known expression vectors can be used. Specifically, a pECCG117, pDZ, pACYC177, pACYC184, pCL, pUC19, pBR322, pMW118, or pCC1BAC vector, or the like may be used.

In an exemplary embodiment, a host cell may introduce a polynucleotide encoding a desired protein in the chromosome by a vector for chromosomal insertion. The introduction of the polynucleotide into the chromosome may be performed by any method known in the art, for example, homologous recombination, but is not limited thereto.

The term "transformation" in the present disclosure means that a recombinant vector containing a polynucleotide encoding a target protein is introduced into a host cell such that the protein encoded by the polynucleotide can be expressed in the host cell. The transformed polynucleotide may be any one as long as the polypeptide can be expressed in the host cell, regardless of whether the polynucleotide is inserted into orpositioned outsidea chromosome.

Additionally, the phrase "operably linked" refers to a functional connection between a promoter sequence, which initiates and mediates the transcription of the polynucleotide encoding the target protein of the present disclosure, and the gene sequence.

Meanwhile, in a specific embodiment of the present disclosure, a microorganism of the genus *Corynebacterium* may further comprise enhanced activities of 3-isopropylmalate dehydrogenase and 3-isopropylmalate dehydratase.

The phrase "enhancement of an activity of a protein" in the present disclosure refers to an activity of the protein enhanced compared with that of a wild-type or pre-modified protein. Specifically, the enhancement of an activity of a protein may include activity increases resulting from improvement of an activity of an endogenous gene encoding the protein, amplification of the endogenous gene by internal or external factors, deletion of a regulatory factor for suppressing the gene expression, an increase in the copy number of a gene, introduction of a foreign gene, modification of an expression regulatory sequence, in particular, replacement or modification of a promoter, and mutation within a gene.

Specifically, the enhancement of an activity of 3-isopropylmalate dehydrogenase or 3-isopropylmalate dehydratase may be performed by a method selected from the group consisting of:
1) increasing the copy number of a polynucleotide encoding said the protein;
2) modifying an expression regulatory sequence to increase the expression of the polynucleotide;
3) modifying the polynucleotide sequence on the chromosome to enhance the activity of the protein; deleting a regulatory factor for suppressing the gene expression; or
4) a combination thereof; however, the method is not particularly limited thereto.

The method of increasing the copy number of the polynucleotide may be performed in the form of operable linkage to a vector, or by insertion into a chromosome in a host cell; however, the method is not particularly limited thereto. Specifically, it may be performed by inserting a vector into a host cell, wherein the vector is operably linked to a polynucleotide encoding the protein of the present disclosure, and can replicate and function regardless of the host cell. Alternatively, it may be performed by a method for increasing the copy number of the polynucleotide inside a chromosome in a host cell by inserting a vector into the host cell, wherein the vector is operably linked to the polynucleotide and enables the polynucleotide to be inserted into the chromosome in the host cell.

The method of modifying an expression regulatory sequence to increase the expression of the polynucleotide may be performed by inducing a mutation on the sequence by deletion, insertion, or non-conservative or conservative substitution of a nucleic acid sequence, or a combination thereof, such that an activity of the expression regulatory sequence can be enhanced, or by replacing the sequence with a nucleic acid sequence having an enhanced activity; however, the method is not particularly limited thereto. The expression regulatory sequence includes a promoter, an operator sequence, a sequence encoding a ribosomal binding site, a sequence regulating the termination of transcription and translation, or the like, but is not particularly limited thereto.

A strong heterologous promoter instead of the original promoter may be linked upstream of a unit for the polynucleotide expression, and examples of the strong promoter may include cj7 promoter (Korean Patent No. 10-0620092 and International Publication No. WO 2006/065095), EF-Tu promoter, groEL promoter, aceA or aceB promoter, and more preferably, cj7 promoter as a *Corynebacterium-*derived promoter is operably linked so that an expression rate of the polynucleotide encoding the protein can be increased.

Additionally, the method of modifying the polynucleotide sequence on the chromosome may be performed by inducing a mutation on the expression regulatory sequence by deletion, insertion, or non-conservative or conservative substitution of a nucleic acid sequence, or a combination thereof, such that an activity of the polynucleotide sequence can be enhanced, or by replacing the sequence with a polynucleotide sequence modified to have an enhanced activity; however, the method is not particularly limited thereto.

The terms "3-isopropylmalate dehydrogenase" and "3-isopropylmalate dehydratase" in the present disclosure refer to enzymes involved in biosynthesis pathways of L-leucine, L-isoleucine, and L-valine. A protein having an activity of the enzymes and a gene encoding the same may include any proteins having an activity of the enzymes and any genes encoding the same without limitation.

Specifically, in the present disclosure, 3-isopropylmalate dehydrogenase (EC1.1.1.85) may be derived from a microorganism of the genus *Corynebacterium,* more specifically, from *Corynebacterium glutamicum.* Meanwhile, 3-isopropylmalate dehydrogenase in the present disclosure may have the amino acid sequence of SEQ ID NO: 17. Further, any protein substantially having an activity of 3-isopropylmalate dehydrogenase as an amino acid sequence substantially showing homology to SEQ ID NO: 17 of 70% or more, specifically 80% or more, more specifically 90% or more, even more specifically 95% or more, and far even more specifically 99% or more may be included in the scope of the present disclosure without limitation.

Further, a gene encoding the 3-isopropylmalate dehydrogenase may have a nucleotide sequence encoding the amino acid sequence of SEQ ID NO: 17. Amino acid sequences of a protein showing the activity are different depending on the species or strain of microorganism, or multiple nucleic acids having an identical function may encode any predetermined proteins because of degeneracy of a genetic code, and accordingly, the gene is not limited to the disclosed SEQ ID NOS. For example, the gene may have a nucleotide sequence of SEQ ID NO: 18, or a nucleotide sequence having homology to SEQ ID NO: 18 of 80%, specifically 90% or more, and more specifically 99% or more.

Additionally, 3-isopropylmalate dehydratase (EC4.2.1.33) in the present disclosure may be derived from a microorganism of the genus *Corynebacterium,* more specifically, from *Corynebacterium glutamicum.* Meanwhile, 3-isopropylmalate dehydratase in the present disclosure consists of two small/large subunits which may have the amino acid sequence of SEQ ID NOS: 19 and 21. Further, any proteins substantially having an activity of 3-isopropylmalate dehydratase may be included in the scope of the present disclosure without limitation as long as the protein substantially has an activity of 3-isopropylmalate dehydratase as an amino acid sequence showing homology to SEQ ID NOS: 19 and 21 of 70% or more, specifically 80% or more, more specifically 90% or more, even more specifically 95% or more, and far even more specifically 99% or more.

Additionally, a gene encoding the 3-isopropylmalate dehydratase may have a nucleotide sequence encoding amino acid sequences of SEQ ID NOS: 19 and 21. Amino acid sequences of a protein showing the activity are different depending on the species or strain of microorganism, or multiple nucleic acids having an identical function may encode any predetermined proteins because of degeneracy of a genetic code, and accordingly, the gene is not limited to the SEQ ID NOS. For example, the gene may have nucleotide sequences of SEQ ID NOS: 20 and 22, or nucleotide sequences having homology thereto of 80%, specifically 90% or more, and more specifically 99% or more.

Meanwhile, in a specific embodiment of the present disclosure, a microorganism of the genus *Corynebacterium* may include an enzyme involved in a biosynthesis pathway of L-isoleucine further including an enhanced activity.

In the present disclosure, the term "enzyme involved in a biosynthesis pathway of L-isoleucine" may include aspartate kinase (lysC gene), aspartate-β-semialdehyde dehydrogenase (asd gene), homoserine dehydrogenase (hom gene), homoserine kinase (thrB gene), threonine synthase (thrC gene), threonine dehydratase (ilvA gene), aminotransferase (ilvE gene), or the like, but is not limited thereto.

Meanwhile, in a specific embodiment of the present disclosure, a microorganism of the genus *Corynebacterium* may further include inactivated pyruvate dehydrogenase.

The term "pyruvate dehydrogenase (EC1.2.4.1)"in the present disclosure refers to an enzyme converting pyruvate to acetyl-CoA and CO₂. The present disclosure can prepare a strain with deleted aceE gene encoding the enzyme to increase the provision of acetyl-CoA used as a precursor of the cimA gene.

Specifically, pyruvate dehydrogenase (EC1.2.4.1) in the present disclosure may be derived from a microorganism of the genus *Corynebacterium,* and more specifically, from *Corynebacterium glutamicum.* Meanwhile, pyruvate dehydrogenase in the present disclosure may have the amino acid sequence of SEQ ID NO: 25. Further, any protein substantially having an activity of pyruvate dehydrogenase as an amino acid sequence substantially showing homology to SEQ ID NO: 25 of 70% or more, specifically 80% or more, more specifically 90% or more, even more specifically 95% or more, and far even more specifically 99% or more may be included in the scope of the present disclosure without limitation.

Additionally, a gene encoding the pyruvate dehydrogenase may have a nucleotide sequence encoding the amino acid sequence of SEQ ID NO: 25. Amino acid sequences of a protein showing the activity are different depending on the species or strain of microorganism, or multiple nucleic acids having an identical function may encode any predetermined proteins because of degeneracy of a genetic code, and accordingly, the gene is not limited to the disclosed SEQ ID NOS. For example, the gene may have the nucleotide sequence of SEQ ID NO: 26, or nucleotide sequences having homology thereto of 80%, specifically 90% or more, and more specifically 99% or more.

The term "inactivation" in the present disclosure refers to the cases in which a gene encoding the relevant polypeptide is not expressed, exhibits a decrease in the activity due to incomplete expression, or does not produce the relevant functional polypeptide, even though the gene is expressed.

Additionally, the term refers to a case in which a gene encoding the relevant polypeptide is substantially not expressed due to a significantly low expression level as well as a case in which the gene is completely inactivated. Accordingly, the inactivation of a gene may be complete (knock-out) or partial (e.g., a gene may be a hypomorph expressing a below-normal level or a product of a mutant gene showing a partial reduction in an activity affected by the gene).

Further, the "inactivation" of the relevant polypeptide in the present disclosure includes cases of elimination of an activity of the polypeptide as well as reduction thereof compared with an unmodified strain. Specifically, the inactivation of threonine dehydratase in the present disclosure may be performed by a method selected from the group consisting of:
1) a partial or complete deletion of a polynucleotide encoding the protein;
2) modification of an expression regulatory sequence to decrease the expression of the polynucleotide;
3) modification of the polynucleotide sequence on the chromosome to attenuate the activity of the protein; or
4) a combination thereof; however, the method is not particularly limited thereto.

The method of a partial or complete deletion of a polynucleotide encoding the protein may be performed by replacing a polynucleotide encoding an endogenous target protein in a chromosome with a marker gene or a polynucleotide in which a nucleic acid sequence was partially deleted, through a vector for chromosomal insertion into a bacterium. As used herein, the term "partially" may vary depending on types of polynucleotide, but may specifically refer to 1 to 300 nucleotides, more specifically, 1 to 100 nucleotides, and even more specifically, 1 to 50 nucleotides.

The method of modification of an expression regulatory sequence to decrease the expression of the polynucleotide may be performed by inducing a mutation on the sequence by deletion, insertion, or non-conservative or conservative substitution of a nucleic acid sequence, or a combination thereof, such that an activity of the expression regulatory sequence can be attenuated, or by replacing the sequence with a nucleic acid sequence having an attenuated activity; however, the method is not particularly limited thereto. The expression regulatory sequence includes a promoter, an operator sequence, a sequence encoding a ribosomal binding site, a sequence regulating the termination of transcription and translation, or the like; but is not limited thereto.

Further, the method of modification of the polynucleotide sequence on the chromosome may be performed by inducing a mutation on the sequence by deletion, insertion, or non-conservative or conservative substitution of a polynucleotide sequence, or a combination thereof, or by replacing the sequence with a polynucleotide sequence modified to have an attenuated activity; however, the method is not limited thereto.

In another specific embodiment of the present disclosure, any microorganism as a microorganism for producing L-isoleucine may be included in the scope of the present disclosure without limitation, as long as the microorganism can be expressed by introducing a protein having an activity of citramalate synthase. Examples of the microorganism may be a microorganism of the genus *Escherichia, Shigella, Citrobacter, Salmonella, Enterobacter, Yersinia, Klebsiella, Erwinia, Corynebacterium, Brevibacterium, Lactobacillus, Selenomanas, Vibrio, Pseudomonas, Streptomyces, Arcanobacterium, Alcaligenes,* or the like, and specifically the genus *Corynebacterium,* more specifically, *Corynebacterium glutamicum,* but are not limited thereto.

Still another aspect of the present disclosure provides a method for producing L-isoleucine comprising
culturing a microorganism of the genus *Corynebacterium* comprising a protein having an activity of citramalate synthase in a medium; and
recovering L-isoleucine from the microorganism or medium,
wherein a microorganism of the genus *Corynebacterium* having an introduced gene encoding a protein having an activity of citramalate synthase is as described above.

Briefly, the microorganism of the genus *Corynebacterium* may have an introduced gene encoding citramalate synthase derived from *Methanocaldococcus,* wherein the citramalate synthase may specifically have an amino acid sequence selected from the group consisting of SEQ IDNOS: 1, 3, 5, 7, 9, 11, 13, and 15.

Additionally, the microorganism of the genus *Corynebacterium* in the present disclosure may further include enhanced activities of 3-isopropylmalate dehydrogenase and 3-isopropylmalate dehydratase. Further, the microorganism of the genus *Corynebacterium* may further include inactivated pyruvate dehydrogenase.

Specifically, a microorganism of the genus *Corynebacterium* in an example of the present disclosure may be *Corynebacterium glutamicum* having an introduced gene encoding citramalate synthase.

In the method, culturing a microorganism of the genus *Corynebacterium* according to the present disclosure may be performed by a well-known batch culture, continuous culture, and fed-batch culture, but is not particularly limited thereto.

In particular, for the culture conditions, an appropriate pH (*e.g.,* a pH of 5 to 9, and specifically, a pH of 6 to 8) may be adjusted using a basic compound (*e.g*., sodium hydroxide, potassium hydroxide, or ammonia) or an acidic compound (*e.g*., phosphoric acid or sulfuric acid), but is not particularly limited thereto. In addition, bubble formation may be inhibited using an antifoaming agent, such as fatty acid polyglycol ester.

Oxygen or an oxygen-containing gas mixture may be introduced into the culture to maintain aerobic conditions, and the temperature of the culture is usually 20°C to 45°C, specifically, 25°C to 40°C. The cultivation continues until the maximum amount of L-isoleucine produced is obtained, and may usually take 10 hours to 160 hours to achieve such objective. L-Isoleucine may be secreted into the medium or may remain in the cells.

Further, as a carbon source for a culture medium used, sugars and carbohydrates (*e.g.,* glucose, sucrose, lactose, fructose, maltose, molasses, starch, and cellulose), oils and fats (*e.g*., soybean oil, sunflower seed oil, peanut oil, and coconut oil), fatty acids (*e.g.*, palmitic acid, stearic acid, and linoleic acid), alcohols (*e.g.,* glycerol and ethanol), organic acids (*e.g.,* acetic acid), or the like may be used individually or in combination, but the carbon source is not limited thereto.

As a nitrogen source, nitrogen-containing organic compounds (*e.g*., peptone, yeast extract, meat juice, malt extract, corn precipitate, soybean meal powder, and urea), inorganic compounds (*e.g*., ammonium sulfate, ammonium chloride, ammonium phosphate, ammonium carbonate, and ammonium nitrate), or the like may be used individually or in combination, but the nitrogen source is not limited thereto.

As a phosphorous source, potassium dihydrogen phosphate, dipotassium hydrogen phosphate, sodium-containing salts corresponding thereto, or the like may be used individually or in combination, but the phosphorous source is not limited thereto.

In a specific embodiment of the present disclosure, upon culturing a microorganism of the genus *Corynebacterium* in the method for producing L-isoleucine, acetate may be further provided.

For a method for recovering L-isoleucine produced by culturing the microorganism in the method, an appropriate method may be used to recover the target amino acid from the culture fluid depending on a culturing method known in the art, *e.g*., batch culture, continuous culture, fed-batch culture, *etc.* For example, centrifugation, filtration, anion exchange chromatography, crystallization, HPLC, or the like may be used, but a method for recovering L-isoleucine is not limited thereto.

Still another aspect of the present disclosure provides the use of a microorganism of the genus *Corynebacterium* comprising a protein having an activity of citramalate synthase for producing L-isoleucine.

In the use, the microorganism of the genus *Corynebacterium* having an introduced gene encoding a protein having an activity of citramalate synthase is as described above.

As described above, the microorganism of the genus *Corynebacterium* for producing L-isoleucine of the present disclosure can produce a high yield of L-isoleucine through a novel biosynthesis pathway which does not use L-threonine as a precursor by having an activity of citramalate synthase introduced.

### [Detailed Description of Embodiment]

### Example 1: Construction of a recombinant vector including cimA gene derived from Methanocaldococcus

### <1-1> Preparation of a fragment of cimA gene derived from Methanocaldococcus

To obtain a 1476 bp fragment comprising the open reading frame (ORF) of cimA gene (NC_000909.1) encoding citramalate synthase, genomic DNA of *Methanocaldococcus jannaschii* DSM 2661was extracted using a Genomic-tip system (Qiagen).

Citramalate synthase derived from *Methanocaldococcus jannaschii* DSM 2661 has the sequence of 491 amino acids represented by SEQ ID NO: 1, and cimA gene encoding the same has the nucleotide sequence of SEQ ID NO: 2.

Polymerase chain reaction (hereinafter abbreviated as "PCR") was performed using the extracted genomic DNA (gDNA) as a template. The PCR was performed using the primer pair of SEQ ID NOS: 35 and 36 under the following conditions: 30 cycles, each consisting of denaturation at 95°C for 30 sec, annealing at 56°C for 30 sec, and elongation at 72°C for 60 sec.

The amplified PCR product was electrophoresed on 1.0% agarose gel, then a band having a desired size was eluted for recovery, and the product was labeled as "cimA(M) fragment".
primer cimA-5-NdeI (SEQ ID NO: 35):
   5'-GCATCATATGATGGTAAGGATATTC-3'
primer cimA-3-XbaI (SEQ ID NO: 36):
   5'-CGATCTAGATTAATTCAACAACATGTT-3'

### <1-2> Construction of recombinant vector p117-cj7-cimA(M)

PCR was performed using p117-cj7-gfp including promoter cj7 derived from a known microorganism of the genus *Corynebacterium* (Korean Patent No. 10-0620092) as a template. As used herein, the term "p117"represents pECCG117, which is an *E. coli-Corynebacterium* shuttle vector (Biotechnology Letters 13(10): 721-726, 1991). The PCR was performed using the primer pair of SEQ ID NOS: 27 and 28 under the following conditions: 30 cycles, each consisting of denaturation at 95°C for 30 sec, annealing at 56°C for 20 sec, and elongation at 72°C for 30 sec.

The amplified PCR product was electrophoresed on 1.0% agarose gel, then a 323 bp band was eluted for recovery, and the product was labeled as "cj7 fragment".
primer Pcj7-5-KpnI (SEQ ID NO: 27):
   5'-GATGGTACCACCCCAGAAACATCCCAGC-3'
primer Pcj7-3 NdeI (SEQ ID NO: 28):
   5'-CGATCATATGGAGTGTTTCCTTTCGTTGGG-3'

Sewing PCR was performed using the prepared cj7 fragment and cimA(M) fragment prepared in Example <1-1> above as a template. The sewing PCR was performed using the primer pair of SEQ ID NOS: 27 and 36 under the following conditions: 30 cycles, each consisting of denaturation at 95°C for 30 sec, annealing at 56°C for 30 sec, and elongation at 72°C for 60 sec.

The amplified PCR product was electrophoresed on 1.0% agarose gel, then a 1799 bp band was eluted for recovery, and the product was labeled as "cj7-cimA(M) fragment".

The obtained cj7-cimA(M) fragment was treated with restriction enzymes KpnI and XbaI, and then ligated with a linear p117 fragment treated with the same restriction enzymes.

*E. coli* DH5α cells were heat-shock transformed with the constructed vector, then plated on a 25 µg/mL kanamycin-containing LB solid medium, and cultured overnight at 37°C. One platinum loop of the cultured colony was inoculated into 3 mL of a 25 µg/mL kanamycin-containing LB liquid medium and cultured overnight, and then plasmid DNA was recovered using a plasmid miniprep kit (Catalogue No. 27104, Qiagen, hereinafter the same).

The construction of the recombinant vector was confirmed by treatment with restriction enzymes KpnI and XbaI, and the clone was identified by performing PCR using the primer pair of SEQ ID NOS: 29 and 30 under the following conditions: 30 cycles, each consisting of denaturation at 95°C for 30 sec, annealing at 56°C for 30 sec, and elongation at 72°C for 90 sec. The recovered recombinant vector was labeled as "p117-cj7-cimA(M)".
primer 117-F (SEQ ID NO: 29):
   5'-CCACAGCCGACAGGATGGTGA-3'
primer 117-R (SEQ ID NO: 30):
   5'-CTCAGGGTGTAGCGGTTCGGT-3'

### <1-3> Preparation of a fragment of leuBCD gene involved in the biosynthesis pathway of 2-ketobutyrate

To prepare a recombinant vector for enhancing a gene in the biosynthesis pathway of 2-ketobutyrate, a fragment of the leuBCD gene encoding 3-isopropylmalate dehydrogenase and 3-isopropylmalate dehydratase derived from a microorganism of the genus *Corynebacterium* was prepared as described below.

To obtain a 1359 bp fragment comprising the ORF of leuB gene encoding 3-isopropylmalate dehydrogenase (EC1.1.1.85), genomic DNA of *Corynebacterium glutamicum* ATCC13032 was extracted using a Genomic-tip system (Qiagen).

3-Isopropylmalate dehydrogenase derived from *Corynebacterium glutamicum* ATCC13032 has the sequence of 340 amino acids represented by of SEQ ID NO: 17, and leuB gene encoding the same has the nucleotide sequence of SEQ ID NO: 18.

PCR was performed using the extracted gDNA as a template. The PCR was performed using the primer pair of SEQ ID NOS: 31 and 32 under the following conditions: 30 cycles, each consisting of denaturation at 95°C for 30 sec, annealing at 56°C for 30 sec, and elongation at 72°C for 60 sec.

The amplified PCR product was electrophoresed on 1.0% agarose gel, then a band having a desired size was eluted for recovery, and the product was labeled as "leuB fragment".
primer leuB-5-cimA (SEQ ID NO: 31):
   5'-TTGTTGAATTAATCTAGAGGTGACACCCCAGTGG-3'
primer leuB-3-leuC (SEQ ID NO: 32):
   5'-TCGCAGCTGCCACCGATATTTAGCTTTGCAGCGC-3'

To obtain a 2078 bp fragment comprising the ORF of leuCD gene encoding 3-isopropylmalate dehydratase (EC4.2.1.33), PCR was performed using gDNA of *Corynebacterium glutamicum* ATCC13032 as a template.

LeuC gene encodes a large subunit of 3-isopropylmalate dehydratase, and the leuC gene derived from *Corynebacterium glutamicum* ATCC13032 has the nucleotide sequence of SEQ ID NO: 20 encoding a polypeptide having the amino acid sequence of SEQ ID NO: 19. Further, leuD gene encodes a small subunit of 3-isopropylmalate dehydratase, and the leuD gene derived from *Corynebacterium glutamicum* ATCC13032 has the nucleotide sequence of SEQ ID NO: 22 encoding a polypeptide having the amino acid sequence of SEQ ID NO: 21.

The PCR was performed using the primer pair of SEQ ID NOS: 33 and 34 under the following conditions: 30 cycles, each consisting of denaturation at 95°C for 30 sec, annealing at 56°C for 30 sec, and elongation at 72°C for 80 sec.

The amplified PCR product was electrophoresed on 1.0% agarose gel, then a band having a desired size was eluted for recovery, and the product was labeled as "leuCD fragment".
primer leuC-5-leuB (SEQ ID NO: 33):
   5'-GCGCTGCAAAGCTAAATATCGGTGGCAGCTGCGA-3'
primer leuD-3-XbaI (SEQ ID NO: 34):
   5'-TGGCGGCCGCTCTAGAGCTTTCGCTATCAGACTG-3'

Sewing PCR was performed using the leuB fragment and leuCD fragment recovered above as a template. The sewing PCR was performed using the primer pair of SEQ ID NOS: 31 and 34 under the following conditions: 30 cycles, each consisting of denaturation at 95°C for 30 sec, annealing at 56°C for 30 sec, and elongation at 72°C for 120 sec.

The amplified PCR product was electrophoresed on 1.0% agarose gel, then a 3437 bp band was eluted for recovery, and the product was labeled as "leuBCD fragment".

### <1-4> Construction of recombinant vector p117-cj7-cimA(M)-leuBCD

To prepare a recombinant vector for enhancing a gene in the biosynthesis pathway of 2-ketobutyrate, p117-cj7-cimA(M), which is the recombinant vector constructed in Example <1-2> above, was treated with restriction enzyme XbaI, the leuBCD fragment recovered in Example <1-3> above was treated with restriction enzyme XbaI, and then both fragments were ligated.

*E. coli* DH5α cells were heat-shock transformed with the constructed vector, then plated on a 25 µg/mL kanamycin-containing LB solid medium, and cultured overnight at 37°C. One platinum loop of the cultured colony was inoculated into 3 mL of a 25 µg/mL kanamycin-containing LB liquid medium and cultured overnight, and then plasmid DNA was recovered using a plasmid miniprep kit.

The construction of the recombinant vector was confirmed by treatment with restriction enzyme XbaI, and the clone was identified by performing PCR using the primer pair of SEQ ID NOS: 29 and 30 under the following conditions: 30 cycles, each consisting of denaturation at 95°C for 30 sec, annealing at 56°C for 30 sec, and elongation at 72°C for 180 sec. The recovered recombinant vector was labeled as "p117-cj7-cimA(M)-leuBCD".

### <1-5> Preparation of a fragment of a cimA(M)mutant using error-prone PCR

To secure a DNA pool having a random mutation introduced to cimA gene, PCR was performed using the cimA(M) fragment recovered in Example <1-1> above as a template and the diversify PCR random mutagenesis kit (Catalogue No. 630703, Clonetech). The PCR was performed on condition 4 of the mutagenesis reaction in Table III in the user manual of the product using the primer pair of SEQ ID NOS: 35 and 36 under the following conditions: 25 cycles, each consisting of denaturation at 95°C for 30 sec and elongation at 68°C for 30 sec.

As a result of the PCR, a mutated art cimA DNA pool having a modification of nucleotide substitution randomly introduced was prepared. The PCR product (hereinafter, referred to as "cimA(M)m fragment") was electrophoresed on 1.0% agarose gel then eluted for recovery.
primer cimA-5-NdeI (SEQ ID NO: 35):
   5'-GCATCATATGATGGTAAGGATATTC-3'
primer cimA-3-XbaI (SEQ ID NO: 36):
   5'-CGATCTAGATTAATTCAACAACATGTT-3'

### <1-6> Construction of mutant library of p117-cj7-cimA(M)m

Recombinant vector, p117-cj7-cimA(M)m, constructed in Example <1-2> above, was treated with restriction enzymes NdeI and XbaI, and was ligated together with the cimA(M)m fragment recovered in Example <1-5> above and treated with the same restriction enzymes.

*E. coli* DH5α cells were heat-shock transformed with the constructed vector, then plated on a 25 µg/mL kanamycin-containing LB solid medium, and cultured overnight at 37°C.The cultured colonies were gathered, and then plasmid DNA was recovered using a plasmid miniprep kit thereby construct the "mutant library of p117-cj7-cimA(M)m".

### Example 2: Confirmation of L-isoleucine producing ability by introduction of cimA gene

To confirm whether *Corynebacterium glutamicum* can has L-isoleucine producing ability when cimA gene derived from *Methanocaldococcus* is introduced thereto, a strain having ilvA gene removed encoding threonine dehydratase was constructed, and recovery of the L-isoleucine producing ability of the strain was examined by introducing the cimA gene thereto.

Threonine dehydratase is an enzyme converting L-threonine, which is a precursor of L-isoleucine biosynthesis, to 2-ketobutyrate. Threonine dehydratase derived from *Corynebacterium glutamicum*ATCC13032 has the amino acid sequence of SEQ ID NO: 23, and ilvA gene encoding the same has the nucleotide sequence of SEQ ID NO: 24. Further, any protein having an activity of threonine dehydratase is included in the scope of the present disclosure without limitation.

### <2-1> Construction of ilvA gene-deleted vector

To obtain an ilvA-gene fragment, PCR was performed using genomic DNA extracted from *Corynebacterium glutamicum* ATCC13032 using a Genomic-tip system (Qiagen) as a template. The PCR was performed using each primer pair of SEQ ID NOS: 37 and 38, and SEQ ID NOS: 39 and 40 under the following conditions: 30 cycles, each consisting of denaturation at 95°C for 30 sec, annealing at 56°C for 30 sec, and elongation at 72°C for 45 sec.

The amplified PCR products were electrophoresed on 1.0% agarose gel, and then 547 bp and 467 bp bands for each PCR were eluted for recovery.

Sewing PCR was performed using the two fragments of the ilvA gene recovered above as a template. The sewing PCR was performed using the primer pair of SEQ ID NOS: 37 and 40 under the following conditions: 30 cycles, each consisting of denaturation at 95°C for 30 sec, annealing at 56°C for 30 sec, and elongation at 72°C for 60 sec.

The amplified PCR product was electrophoresed on 1.0% agarose gel, then a 1014 bp band was eluted for recovery, and the recovered ilvA gene-deleted fragment was labeled as "DilvA".

The DilvA fragment recovered above was treated with restriction enzyme XbaI, pDZ vector (Korean Patent No. 10-0924065) was treated with same restriction enzyme to recover a linear pDZ fragment, and then the DilvA and linear pDZ fragments were ligated to construct a recombinant vector having ilvA gene deleted.

*E. coli* DH5α cells were heat-shock transformed with the constructed recombinant vector, then plated on a 25 µg/mL kanamycin-containing LB solid medium, and cultured overnight at 37°C. One platinum loop of the cultured colony was inoculated into 3 mL of a 25 µg/mL kanamycin-containing LB liquid medium and cultured overnight, and then plasmid DNA was recovered using a plasmid miniprep kit.

The construction of the recombinant vector was confirmed by treatment with restriction enzyme XbaI, and the clone was identified by performing PCR using the primer pair of SEQ ID NOS: 41 and 42 under the following conditions: 30 cycles, each consisting of denaturation at 95°C for 30 sec, annealing at 56°C for 30 sec, and elongation at 72°C for 90 sec. The recovered recombinant vector was labeled as "pDZ-ilvA(Del)".
primer ilvA-5-XbaI (SEQ ID NO: 37):
   5'-GCATCTAGAGAACACGGACAATGCCAC-3'
primer ilvA-Del-R (SEQ ID NO: 38):
   5'-CAAACAGCGTGATGTCCTGAGTGAGCTGCGCT-3'
primer ilvA-Del-F (SEQ ID NO: 39):
   5'-AGCGCAGCTCACTCAGGACATCACGCTGTTTG-3'
primer ilvA-3-XbaI (SEQ ID NO: 40):
   5'-GCATCTAGAACCTGCGGCACACCTTGGGC-3'
primer Topo-F (SEQ ID NO: 41):
   5'-GCAGTGAGCGCAACGCAAT-3'
primer Topo-R (SEQ ID NO: 42):
   5'-CGTTGTAAAACGACGGCCA-3'

### <2-2> Construction of ilvA gene-deleted strain

The recombinant vector pDZ-ilvA(Del) constructed in Example <2-1> above was introduced into *Corynebacterium glutamicum* ATCC13032 as a parent strain by electroporation, and was plated on a solid medium containing 25 µg/mL of kanamycin to select a single colony. From the colony, each strain having the pDZ-ilvA(Del) vector introduced into a chromosome was selected by the second passage, and then PCR was performed using gDNA recovered from each of the selected strains as a template. The PCR was performed using the primer pair of SEQ ID NOS: 43 and 44 under the following conditions: 30 cycles, each consisting of denaturation at 95°C for 30 sec, annealing at 56°C for 30 sec, and elongation at 72°C for 90 sec.

As a result of the PCR, an ilvA gene-deleted strain was recovered, and was labeled as an "ATCC13032ΔilvA" strain.
primer CFilvA (SEQ ID NO: 43):
   5'-GATCTGTGATGAGGTGATG-3'
primer CRilvA (SEQ ID NO: 44):
   5'-CGTCCTTCCATGACTCTCA-3'

### <2-3> Preparation of a gene fragment involved in the biosynthesis pathway of L-isoleucine

To prepare a recombinant vector for enhancing a gene in the biosynthesis pathway of L-isoleucine, fragments of lysC, asd, hom, thrB, thrC, and ilvA genes derived from a microorganism of the genus *Corynebacterium* were prepared as described below.

First, to obtain a DNA fragment comprising the ORF of lysC-asd gene encoding aspartate kinase and aspartate-β-semialdehyde dehydrogenase, PCR was performed using genomic DNA extracted from *Corynebacterium glutamicum* ATCC13032 as a template as described in Example <2-1>. The PCR was performed using the primer pair of SEQ ID NOS: 45 and 46 under the following conditions: 30 cycles, each consisting of denaturation at 95°C for 30 sec, annealing at 56°C for 30 sec, and elongation at 72°C for 90 sec.

The amplified PCR product was electrophoresed on 1.0% agarose gel, then a 2666 bp band was eluted for recovery, and the product was labeled as "lysC-asd fragment".
primer lysC-5-KpnI (SEQ ID NO: 45):
   5'-TGAGGTACCCATGCGATTGTTAATGC-3'
primer asd-3-SfoI (SEQ ID NO: 46):
   5'-CTAGGCGCCAGTGTAGCACTCAAGCGGA-3'

To obtain a DNA fragment comprising the ORF of hom-thrB gene encoding homoserine dehydrogenase and homoserine kinase, PCR was performed using genomic DNA extracted from *Corynebacterium glutamicum* ATCC13032 as a template as described in Example <2-1>. The PCR was performed using the primer pair of SEQ ID NOS: 47 and 48 under the following conditions: 30 cycles, each consisting of denaturation at 95°C for 30 sec, annealing at 56°C for 30 sec, and elongation at 72°C for 90 sec.

The amplified PCR product was electrophoresed on 1.0% agarose gel, then a 2633 bp band was eluted for recovery, and the product was labeled as "hom-thrB fragment".
primer hom-5-BamHI (SEQ ID NO: 47):
   5'-CTAGGATCCCTCACCATTCTCAATGGT-3'
primer thrB-3-BamHI (SEQ ID NO: 48):
   5'-CTAGGATCCGCCTTCCTTGTTGGGC-3'

To obtain a DNA fragment comprising the ORF of thrC gene encoding threonine synthase, PCR was performed using genomic DNA extracted from *Corynebacterium glutamicum* ATCC13032 as a template as described in Example <2-1>. The PCR was performed using the primer pair of SEQ ID NOS: 49 and 50 under the following conditions: 30 cycles, each consisting of denaturation at 95°C for 30 sec, annealing at 56°C for 30 sec, and elongation at 72°C for 60 sec.

The amplified PCR product was electrophoresed on 1.0% agarose gel, then a 1703 bp band was eluted for recovery, and the product was labeled as "thrC fragment".
primer thrC-5-SpeI (SEQ ID NO: 49):
   5'-TGCACTAGTGAGAACATACAGGTTCCA-3'
primer thrC-3-ilvA (SEQ ID NO: 50):
   5'-GGCATTGTCCGTGTTCTTACTTCACGGAAGTG-3'

To obtain a DNA fragment comprising the ORF of ilvA gene encoding threonine dehydratase, PCR was performed using genomic DNA extracted from *Corynebacterium glutamicum* ATCC13032 as a template as described in Example <2-1>. The PCR was performed using the primer pair of SEQ ID NOS: 51 and 52 under the following conditions: 30 cycles, each consisting of denaturation at 95°C for 30 sec, annealing at 56°C for 30 sec, and elongation at 72°C for 60 sec.

The amplified PCR product was electrophoresed on 1.0% agarose gel, then a 1862 bp band was eluted for recovery, and the product was labeled as "ilvA fragment".
primer ilvA-5-thrC (SEQ ID NO: 51):
   5'-CACTTCCGTGAAGTAAGAACACGGACAATGCC-3'
primer ilvA-3-SpeI (SEQ ID NO: 52):
   5'-TGCACTAGTACCTGCGGCACACCTTGGGC-3'

Sewing PCR was performed using the thrC fragment and ilvA fragment recovered above as a template. The sewing PCR was performed using the primer pair of SEQ ID NOS: 49 and 52 under the following conditions: 30 cycles, each consisting of denaturation at 95°C for 30 sec, annealing at 56°C for 30 sec, and elongation at 72°C for 120 sec.

The amplified PCR product was electrophoresed on 1.0% agarose gel, then a 3565 bp band was eluted for recovery, and the product was labeled as "thrC-ilvA fragment".

### <2-4> Construction of a vector for enhancing a gene in the biosynthesis pathway of L-isoleucine

To prepare a recombinant vector for enhancing a gene in the biosynthesis pathway of L-isoleucine, the lysC-asd fragment obtained in Example <2-3> above was treated with restriction enzymes KpnI and SfoI, and then ligated with a linear p117 fragment treated with restriction enzymes KpnI and EcoRV.

A plasmid DNA was recovered in the same manner of Example <1-2> above. To confirm the construction of the recombinant vector, the clone was identified by performing PCR using the primer pair of SEQ ID NOS: 29 and 30 under the following conditions: 30 cycles, each consisting of denaturation at 95°C for 30 sec, annealing at 56°C for 30 sec, and elongation at 72°C for 100 sec. The recovered recombinant vector was labeled as "p117-lysC-asd".

The hom-thrB fragment obtained in Example <2-3> above was treated with restriction enzyme BamHI, and then ligated with a linear p117-lysC-asd fragment treated with the same restriction enzyme.

A plasmid DNA was recovered in the same manner of Example <1-2> above. To confirm the construction of the recombinant vector, the clone was identified by performing PCR using the primer pair of SEQ ID NOS: 29 and 30 under the following conditions: 30 cycles, each consisting of denaturation at 95°C for 30 sec, annealing at 56°C for 30 sec, and elongation at 72°C for 180 sec. The recovered recombinant vector was labeled as "p117-lysC-asd-hom-thrB".

The thrC-ilvA fragment obtained in Example <2-3> above was treated with restriction enzyme SpeI, and then ligated with a linear p117-lysC-asd-hom-thrB fragment treated with the same restriction enzyme.

A plasmid DNA was recovered in the same manner of Example <1-2> above. To confirm the construction of the recombinant vector, the clone was identified by performing PCR using the primer pair of SEQ ID NOS: 29 and 30 under the following conditions: 30 cycles, each consisting of denaturation at 95°C for 30 sec, annealing at 56°C for 30 sec, and elongation at 72°C for 300 sec.

The recovered recombinant vector was labeled as "p117-IBGC (Isoleucine Biosynthesis Genes Cluster)", which is a recombinant vector for enhancing a gene in the biosynthesis pathway of L-isoleucine including fragments of lysC, asd, hom, thrB, thrC, and ilvA genes, six genes involved in the biosynthesis of L-isoleucine.

### <2-5> Comparison of L-isoleucine productivity

Recombinant vectors p117-cj7-cimA(M), p117-cj7-cimA(M)-leuBCD, and p117-IBGC constructed in Examples <1-2>, <1-4>, and <2-4> above were introduced into *Corynebacterium glutamicum* ATCC13032 by electroporation, and were plated on a solid medium containing 25 µg/mL of kanamycin to select a single colony. From the colony, each of the selected strains was labeled as *Corynebacterium glutamicum* "ATCC13032/p117-cj7-cimA(M)", "ATCC13032/p117-cj7-cimA(M)-leuBCD", and "ATCC13032/p117-IBGC".

Further, p117-cj7-cimA(M) and p117-cj7-cimA(M)-leuBCD were introduced into a *Corynebacterium glutamicum* ATCC13032ΔilvA strain prepared in Example <2-2> above by electroporation, and were plated on a solid medium containing 25 µg/mL of kanamycin to select a single colony. From the colony, each of the selected strains was labeled as *Corynebacterium glutamicum* "ATCC13032ΔilvA/p117-cj7-cimA(M)" and "ATCC13032ΔilvA/p117-cj7-cimA(M)-leuBCD".

Titer evaluation on L-isoleucine productivity was performed using *Corynebacterium glutamicum* ATCC13032, ATCC13032/p117-IBGC, ATCC13032/pl 17-cj7-cimA(M), ATCC13032/p117-cj7-cimA(M)-leuBCD, ATCC13032ΔilvA, ATCC13032ΔilvA/p117-cj7-cimA(M), and ATCC13032ΔilvA/p117-cj7-cimA(M)-leuBCD.

Specifically, each of the strains was inoculated into a 250 mL flask containing 25 mL of a glucose-containing titer medium having the composition shown in Table 1 below, and was then cultured in an incubator at 32°C and 200 rpm for 30 hours. The concentrations of L-isoleucine produced are shown in Table 2 below.

**[Table 1]**

| Composition | Concentration (per liter) |
|---|---|
| Glucose | 60 g |
| KH₂PO₄ | 1.1 g |
| (NH₄)₂SO₄ | 20 g |
| MgSO₄·7H₂O | 1.2 g |
| FeSO₄·7H₂O | 90 mg |
| MnSO₄·4H₂O | 90 mg |
| Thiamine-HCl | 4.5 mg |
| d-Biotin | 0.9 mg |
| HSM | 20g |
| Calcium carbonate | 30 g |
| pH | 7.0 |

**[Table 2]**

| Strain | L-isoleucine (g/L) |
|---|---|
| ATCC13032 | 0.02 |
| ATCC13032/p117-IBGC | 0.70 |
| ATCC13032/p117-cj7-cimA(M) | 0.83 |
| ATCC13032/p117-cj7-cimA(M)-leuBCD | 1.25 |
| ATCC13032ΔilvA | 0.00 |
| ATCC13032ΔilvA/p117-cj 7-cimA(M) | 0.08 |
| ATCC13032ΔilvA/p117-cj7-cimA(M)-leuBCD | 0.15 |

As disclosed in Table 2 above, a wild-type strain ATCC13032 having ilvA gene produced 0.02 g/L of L-isoleucine, and ATCC13032/p117-IBGC, a transformed strain having the genes in the biosynthesis pathway of L-isoleucine concurrently introduced, produced 0.70 g/L of L-isoleucine, showing that L-isoleucine productivity increased by 3400% compared with the wild-type strain.

Further, transformed strains ATCC13032/p117-cj7-cimA(M) and ATCC13032/p117-cj7-cimA(M)-leuBCD produced 0.83 g/L and 1.25 g/L of L-isoleucine, respectively, showing that L-isoleucine productivity increased by 4050% and 6150%, respectively, compared with the wild-type strain.

From the comparison, the L-isoleucine productivity of ATCC13032/p117-cj7-cimA(M), a strain transformed to introduce a cimA gene into the existing biosynthesis pathway of L-isoleucine, was confirmed as being superior to that of ATCC13032/p117-IBGC, a strain transformed to enhance the biosynthesis pathway of L-isoleucine itself. Further, the L-isoleucine productivity of ATCC13032/p117-cj7-cimA(M)-leuBCD, a transformed strain further enhanced with leuBCD gene, was confirmed as being further capable.

Meanwhile, the ilvA gene-deleted ATCC13032ΔilvA strain lost the L-isoleucine productivity, whereas the ATCC13032ΔilvA/p117-cj7-cimA(M) strain having cimA gene introduced produced 0.08 g/L of L-isoleucine, and the ATCC13032ΔilvA/p117-cj7-cimA(M)-leuBCD strain having cimA gene introduced and a leuBCD gene enhanced produced 0.15 g/L thereof.

The above results show that producing L-isoleucine is possible using a novel biosynthesis pathway of L-isoleucine by the introduction of cimA gene, not the existing biosynthesis pathway of L-isoleucine using L-threonine as a precursor.

### Example 3: Preparation of a transformed recombinant strain and comparison of L-isoleucine productivity

### <3-1> Preparation of a recombinant strain using a wild-type microorganism of the genus Corynebacterium

To provide acetyl-CoA used as a precursor of cimA gene, acetate-auxotrophic strain of wild-type *Corynebacterium glutamicum* ATCC13032 in which aceE gene encoding pyruvate dehydrogenase is deleted was prepared, and was labeled as "ATCC13032ΔaceE" (Schreiner et al., J Bacteriol. 187: 6005-18, 2005).

Pyruvate dehydrogenase derived from *Corynebacterium glutamicum* ATCC13032 has the amino acid sequence of SEQ ID NO: 25, and aceE gene encoding the same has the nucleotide sequence of SEQ ID NO: 26.

Recombinant vector p117-cj7-cimA(M) and mutant library of p117-cj7-cimA(M)m prepared in Example 1 above were introduced into the ATCC13032ΔaceE strain prepared above by electroporation. The constructed strains were labeled as
"ATCC13032ΔaceE/p117-cj7-cimA(M)" and "ATCC13032ΔaceE/p117-cj7-cimA(M)m mutant library", respectively.

The ATCC13032ΔaceE, ATCC13032ΔaceE/p117-cj7-cimA(M), and ATCC13032ΔaceE/p117-cj7-cimA(M)m mutant library were plated on a solid medium containing 25 µg/mL of kanamycin to select a single colony, and titer evaluation on L-isoleucine productivity was performed using the selected strains.

Specifically, the strains were inoculated into a 250 mL flask containing 25 mL of a titer medium containing acetate in the form of ammonium acetate having the composition shown in Table 3 below, and then were cultured in an incubator at 32°C and 200 rpm for 30 hours. As a result, seven types of colonies having increased L-isoleucine concentration were selected, and the list and L-isoleucine concentrations thereof are shown in Table 4 below.

**[Table 3]**

| Composition | Concentration (per liter) |
|---|---|
| Glucose | 60 g |
| C₂H₇NO₂ | 5 g |
| KH₂PO₄ | 1.1 g |
| (NH₄)₂SO₄ | 20 g |
| MgSO₄·7H₂O | 1.2 g |
| FeSO₄·7H₂O | 90 mg |
| MnSO₄·4H₂O | 90 mg |
| Thiamine-HCl | 4.5 mg |
| d-Biotin | 0.9 mg |
| HSM | 20g |
| Calcium carbonate | 30 g |
| pH | 7.0 |

**[Table 4]**

| Strain | L-isoleucine (g/L) |
|---|---|
| ATCC13032ΔaceE | 0.05 |
| ATCC13032ΔaceE/p117-cj7-cimA(M) | 0.84 |
| ATCC13032ΔaceE/p117-cj7-cimA(M)m1 | 1.02 |
| ATCC13032ΔaceE/p117-cj7-cimA(M)m2 | 1.35 |
| ATCC13032ΔaceE/p117-cj7-cimA(M)m3 | 2.23 |
| ATCC13032ΔaceE/p117-cj7-cimA(M)m4 | 1.24 |
| ATCC13032ΔaceE/p117-cj7-cimA(M)m5 | 0.96 |
| ATCC13032ΔaceE/p117-cj7-cimA(M)m6 | 1.73 |
| ATCC13032ΔaceE/p117-cj7-cimA(M)m7 | 0.97 |

As a result, as disclosed in Table 4 above, the ATCC13032ΔaceE strain as a parent strain produced 0.05 g/L of L-isoleucine, and transformed strains produced 0.84 g/L to 2.23 g/L of L-isoleucine, showing that the L-isoleucine productivity increased by 1580% to 4360%, respectively, compared with the parent strain. In particular, transformed strain ATCC13032ΔaceE/p117-cj7-cimA(M)m3 recorded the highest L-isoleucine productivity among the strains compared with the parent strain ATCC13032ΔaceE.

To identify the mutation site of the mutants of cimA(M) gene selected by the increased productivity of L-isoleucine shown in Table 4, and the substituted amino acids of each mutation, sequencing was performed.

As a result, as the CimA(M)m mutant library,
CimA(M)m1 has the amino acid sequence of SEQ ID NO: 3, and may be encoded by a polynucleotide having the nucleotide sequence of SEQ ID NO: 4;
CimA(M)m2 has the amino acid sequence of SEQ ID NO: 5, and may be encoded by a polynucleotide having the nucleotide sequence of SEQ ID NO: 6;
CimA(M)m3 has the amino acid sequence of SEQ ID NO: 7, and may be encoded by a polynucleotide having the nucleotide sequence of SEQ ID NO: 8;
CimA(M)m4 has the amino acid sequence of SEQ ID NO: 9, and may be encoded by a polynucleotide having the nucleotide sequence of SEQ ID NO: 10;
CimA(M)m5 has the amino acid sequence of SEQ ID NO: 11, and may be encoded by a polynucleotide having the nucleotide sequence of SEQ ID NO: 12;
CimA(M)m6 has the amino acid sequence of SEQ ID NO: 13, and may be encoded by a polynucleotide having the nucleotide sequence of SEQ ID NO: 14; and
CimA(M)m7 has the amino acid sequence of SEQ ID NO: 15, and may be encoded by a polynucleotide having the nucleotide sequence of SEQ ID NO: 16.

### <3-2> Preparation of a recombinant strain using p117-cj7-cimA(M)m3 having the highest activity of the selected p117-cimA(M)m mutant library

The p117-cj7-cimA(M)m3 vector identified as having the highest L-isoleucine productivity in Example <3-1> above was treated with XbaI, and was ligated with a DNA fragment recovered by treating a leuBCD fragment recovered in Example <1-3> above with the same restriction enzyme.

*E. coli* DH5α cells were heat-shock transformed with the constructed vector, then plated on a 25 µg/mL kanamycin-containing LB solid medium, and cultured overnight at 37°C. One platinum loop of the cultured colony was inoculated into 3 mL of a 25 µg/mL kanamycin-containing LB liquid medium and cultured overnight, and then plasmid DNA was recovered using a plasmid miniprep kit.

The construction of the recombinant vector was confirmed by treatment with restriction enzyme XbaI, and the clone was identified by performing PCR using the primer pair of SEQ ID NOS: 29 and 30 under the following conditions: 30 cycles, each consisting of denaturation at 95°C for 30 sec, annealing at 56°C for 30 sec, and elongation at 72°C for 180 sec. The recovered recombinant vector was labeled as "p117-cj7-cimA(M)m3-leuBCD".

Each ofl7-cj7-cimA(M) of Example <1-2>, p117-cj7-cimA(M)-leuBCD of Example <1-4>, p117-cj7-cimA(M)m3 of Example <3-1>, and p117-cj7-cimA(M)m3-leuBCD of Example <3-2> above was introduced into the ATCC13032ΔaceE strain used in Example <3-1> by electroporation, and was plated on a solid medium containing 25 µg/mL of kanamycin to select a single colony.

The selected strains were inoculated into a 250 mL flask containing 25 mL of a titer medium containing glucose having the composition shown in Table 3 above, and then were cultured in an incubator at 32°C and 200 rpm for 30 hours to examine the L-isoleucine productivity. The results are shown in Table 5 below.

**[Table 5]**

| Strain | L-isoleucine (g/L) |
|---|---|
| ATCC13032ΔaceE | 0.04 |
| ATCC13032ΔaceE/p117-cj7-cimA(M) | 0.98 |
| ATCC13032ΔaceE/p117-cj 7-cimA(M)-leuBCD | 2.09 |
| ATCC13032ΔaceE/p117-cj7-cimA(M)m3 | 2.22 |
| ATCC13032ΔaceE/p117-cj7-cimA(M)m3-IcuBCD | 3.76 |

As disclosed in Table 5 above, while the ATCC13032ΔaceE strain as a parent strain produced 0.04 g/L of L-isoleucine, transformed strain ATCC13032ΔaceE/p117-cj7-cimA(M) produced 0.98 g/L of L-isoleucine and ATCC13032ΔaceE/p117-cj7-cimA(M)-leuBCD produced 2.09 g/L of L-isoleucine, showing increases of 0.94 g/L and 2.05 g/L compared with the L-isoleucine productivity of the parent strain.

Further, transformed strains ATCC13032ΔaceE/p117-cj7-cimA(M)m3 and ATCC13032ΔaceE/p117-cj7-cimA(M)m3-leuBCD including the cimA(M)m3 mutant gene recovered in Example <3-1> produced 2.22 g/L and 3.76 g/L of L-isoleucine, respectively, which increased by 5450% and 9300% of the production compared with the parent strain. Accordingly, this result proves that the cimA(M)m3 mutant is more effective than a wild-type strain thereof in the increase of the L-isoleucine productivity.

Among the transformed strains recovered in the above Examples, ATCC13032ΔaceE/p117-cj7-cimA(M)m3 was labeled as "*Corynebacterium glutamicum* CA10-1002", deposited to the Korean Culture Center of Microorganisms (KCCM) under the Budapest Treaty on February 27, 2015, and was provided with Accession No. KCCM11672P.

### <3-3> Preparation of a recombinant strain using L-isoleucine-producing strain KCCM11248P

Each of p117-cj7-cimA(M), p117-cj7-cimA(M)-leuBCD, p117-cj7-cimA(M)m3, and p117-cj7-cimA(M)m3-leuBCD, the recombinant vectors used in Examples <3-1> and <3-2>, was introduced into *Corynebacterium glutamicum* KCCM11248P, an L-isoleucine-producing strain (Korean Patent No. 10-1335789), and was plated on a solid medium containing 25 µg/mL of kanamycin to select a single colony.

Each of the selected strains was labeled as KCCM11248P/p117-cj7-cimA(M), KCCM11248P/p117-cj7-cimA(M)-leuBCD, KCCM11248P/p117-cj7-cimA(M)m3, and KCCM11248P/p117-cj7-cimA(M)m3-leuBCD.

The L-isoleucine productivity of the strains was examined using L-isoleucine titer media having the composition shown in Table 6 below and culturing the strains in flasks as described below.

**[Table 6]**

| Composition | Concentration (per liter) |
|---|---|
| Glucose | 45 g |
| BM | 10 g |
| KH₂PO₄ | 1.1 g |
| (NH₄)₂SO₄ | 12.5 g |
| MgSO₄·7H₂O | 1.2 g |
| FeSO₄·7H₂O | 180 mg |
| MnSO₄·4H₂O | 180 mg |
| ZnSO₄·5H₂O | 0.9 mg |
| CuSO₄·5H₂O | 0.9 mg |
| Thiamine-HCl | 9 mg |
| d-Biotin | 1.8 mg |
| Ca-Pantothenate | 9 mg |
| NCA | 60 mg |
| HSM | 20 g |
| Calcium carbonate | 30 g |
| pH | 7.0 |

Specifically, parent strain KCCM11248P and transformed strains KCCM11248P/p117-cj7-cimA(M), KCCM11248P/p117-cj7-cimA(M)-leuBCD, KCCM11248P/p117-cj7-cimA(M)m3, and KCCM11248P/p117-cj7-cimA(M)m3-leuBCD were inoculated into 250 mL flasks containing 25 mL of titer media containing glucose having the composition shown in Table 6, and then were cultured in an incubator at 32°C and 200 rpm for 60 hours to examine the L-isoleucine productivity. The results are shown in Table 7 below.

**[Table 7]**

| Strain | L-isoleucine (g/L) |
|---|---|
| KCCM11248P | 3.33 |
| KCCM11248P/p117-cj7-cimA(M) | 4.04 |
| KCCM11248P/p117-cj7-cimA(M)-leuBCD | 4.82 |
| KCCM 11248P/p117-cj7-cimA(M)m3 | 4.71 |
| KCCM11248P/p117-cj7-cimA(M)m3-leuBCD | 11.21 |

As disclosed in Table 7 above, while the parent strain KCCM11248P produced 3.33 g/L of L-isoleucine, transformed strains KCCM11248P/p117-cj7-cimA(M) produced 4.04 g/L of L-isoleucine, and KCCM11248P/p117-cj7-cimA(M)-leuBCD produced 4.82 g/L of L-isoleucine, showing increases of 0.71 g/L and 1.49 g/L compared with the L-isoleucine productivity of the parent strain.

Meanwhile, the transformed strains KCCM11248P/p117-cj7-cimA(M)m3 and KCCM11248P/p117-cj7-cimA(M)m3-leuBCD produced 4.71 g/L and 11.21 g/L of L-isoleucine, respectively, which increased in production by 41.4% and 236.6% compared with the parent strain.

As shown in the result of Example <3-2> using *Corynebacterium glutamicum* ATCC13032, this result further proves that the cimA(M)m3 mutant is more effective than a wild-type strain thereof in the increase of the L-isoleucine productivity.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

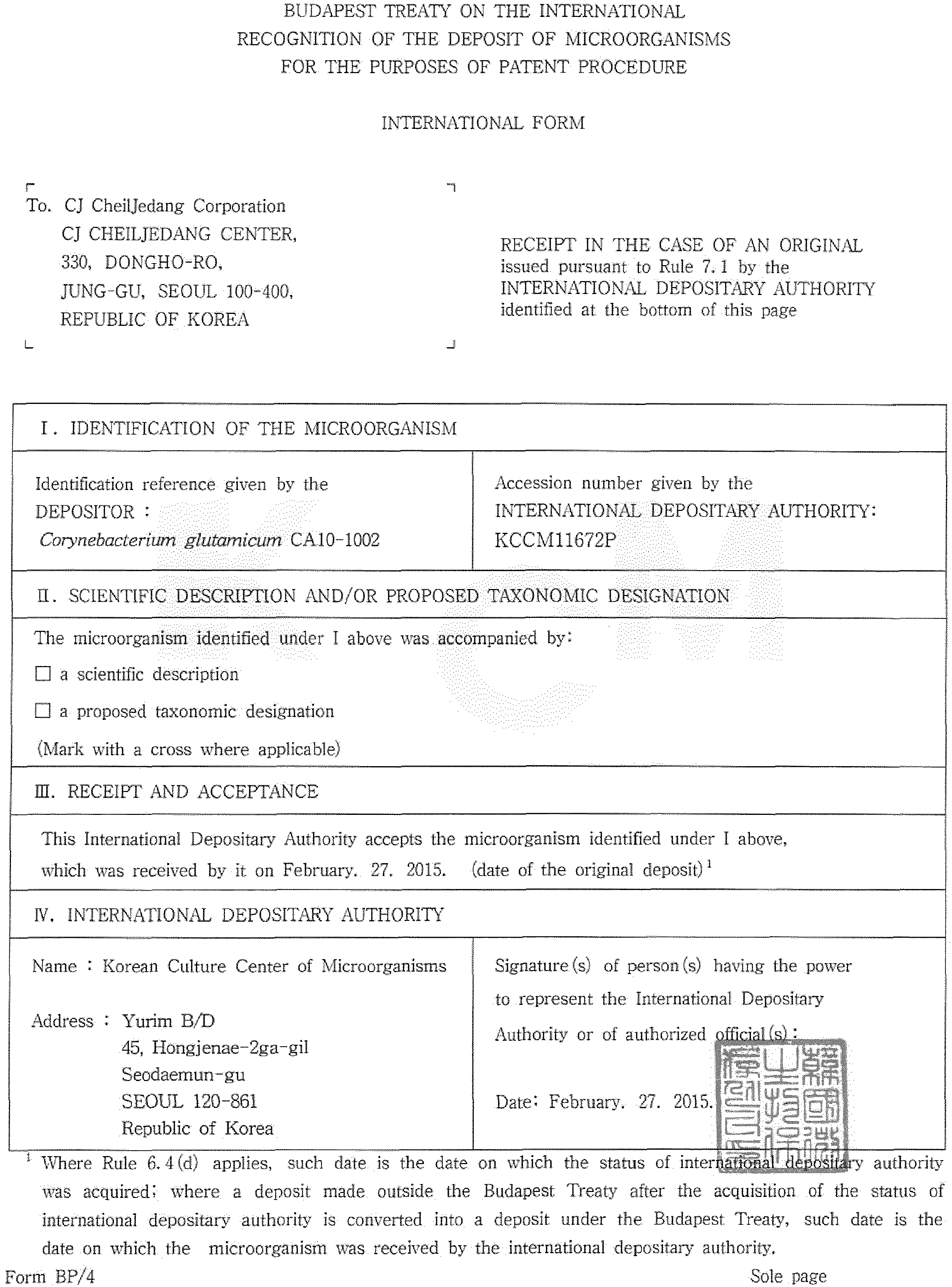
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400,
REPUBLIC OF KOREA

RECEIPT IN THE CASE OF AN ORIGINAL
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR :
Corynebacterium glutamicum CA10-1002 | Accession number given by the
INTERNATIONAL DEPOSITARY AUTHORITY:
KCCM11672P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on February. 27. 2015. (date of the original deposit)¹
IV. INTERNATIONAL DEPOSITARY AUTHORITY
Name : Korean Culture Center of Microorganisms

Address : Yurim B/D
45, Hongjenae-2ga-gil
Seodaemun-gu
SEOUL 120-861
Republic of Korea | Signature(s) of person(s) having the power
to represent the International Depositary
Authority or of authorized official(s):

Date: February. 27. 2015.
¹ Where Rule 6.4(d) applies, such date is the date on which the status of international depositary authority
was acquired; where a deposit made outside the Budapest Treaty after the acquisition of the status of
international depositary authority is converted into a deposit under the Budapest Treaty, such date is the
date on which the microorganism was received by the international depositary authority.
Form BP/4 Sole page

<110>    CJ CHEILJEDANG CORPORATION

<120>    MICROORGANISMS HAVING L-ISOLEUCINE PRODUCTIVITY AND PROCESS FOR
         PRODUCING L-ISOLEUCINE USING THE SAME

<130>    OPA16137-PCT

<150>    KR 10-2015-0148157
<151>    2015-10-23

<160>    52

<170>    KopatentIn 2.0

<210>    1
<211>    491
<212>    PRT
<213>    cimA from Methanocaldococcus jannaschii

<400>    1
Met Met Val Arg Ile Phe Asp Thr Thr Leu Arg Asp Gly Glu Gln Thr
  1               5                  10                  15

Pro Gly Val Ser Leu Thr Pro Asn Asp Lys Leu Glu Ile Ala Lys Lys
             20                  25                  30

Leu Asp Glu Leu Gly Val Asp Val Ile Glu Ala Gly Ser Ala Ile Thr
         35                  40                  45

Ser Lys Gly Glu Arg Glu Gly Ile Lys Leu Ile Thr Lys Glu Gly Leu
     50                  55                  60

Asn Ala Glu Ile Cys Ser Phe Val Arg Ala Leu Pro Val Asp Ile Asp
 65                  70                  75                  80

Ala Ala Leu Glu Cys Asp Val Asp Ser Val His Leu Val Val Pro Thr
                 85                  90                  95

Ser Pro Ile His Met Lys Tyr Lys Leu Arg Lys Thr Glu Asp Glu Val
            100                 105                 110

Leu Glu Thr Ala Leu Lys Ala Val Glu Tyr Ala Lys Glu His Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Thr Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Leu Lys Ile

225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu Val Glu Glu Lys Ile Lys Leu Asp Glu Leu Thr Val
    370                 375                 380

Val Ser Gly Asn Lys Ile Thr Pro Ile Ala Ser Val Lys Leu His Tyr
385                 390                 395                 400

Lys Gly Glu Asp Ile Thr Leu Ile Glu Thr Ala Tyr Gly Val Gly Pro
                405                 410                 415

Val Asp Ala Ala Ile Asn Ala Val Arg Lys Ala Ile Ser Gly Val Ala
            420                 425                 430

Asp Ile Lys Leu Val Glu Tyr Arg Val Glu Ala Ile Gly Gly Gly Thr
        435                 440                 445

Asp Ala Leu Ile Glu Val Val Val Lys Leu Arg Lys Gly Thr Glu Ile
    450                 455                 460

Val Glu Val Arg Lys Ser Asp Ala Asp Ile Ile Arg Ala Ser Val Asp
465                 470                 475                 480

Ala Val Met Glu Gly Ile Asn Met Leu Leu Asn
                485                 490

<210>   2
<211>   1476
<212>   DNA
<213>   cimA from Methanocaldococcus jannaschii

<400>   2
atgatggtaa ggatatttga tacaacactt agagatggag agcaaacacc aggagtttct      60

ttaacaccaa atgataagtt agagatagca aaaaaattgg atgagcttgg agttgatgtt     120

atagaggcag gttcagctat aacttcaaaa ggagagagag aaggaataaa attaataaca     180

aaagaaggtt taaatgcaga aatctgctca tttgttagag ctttacctgt agatattgat 240

gctgccttag aatgtgatgt agatagtgtc catttagtag tgccaacatc tccaatacac 300

atgaaatata agcttagaaa aacagaagat gaggttttag agacagcttt aaaggctgta 360

gagtatgcta aagaacatgg attgattgtt gagttatctg cagaggatgc aacaagaagt 420

gatgtaaatt tcttaataaa actatttaat gaaggggaaa aggttggagc agacagagtt 480

tgtgtttgtg acacagtagg agttttaact ccacaaaaga gtcaggaatt atttaaaaaa 540

ataactgaaa atgttaattt accggtctca gttcattgcc acaacgactt tggaatggct 600

actgctaata cttgctcagc agttttaggt ggagctgttc agtgccacgt aacagttaat 660

ggtattggag agagagcagg aaatgcctca ttggaagagg ttgttgctgc tttaaaaata 720

ctctatggct atgatactaa gataaagatg gaaaagttat atgaggtttc aagaattgtc 780

tcaagattga tgaaacttcc tgttccacca aataaagcaa ttgttgggga caatgcattt 840

gctcatgaag caggaataca tgttgatgga ttaataaaaa atactgaaac ctatgagcca 900

ataaaaccag aaatggttgg gaatagaaga agaattattt tgggtaagca ttctggtaga 960

aaagctttaa aatacaaact tgatttgatg ggcataaacg ttagtgatga gcaattaaat 1020

aaaatatatg aaagagttaa agaatttggg gatttgggta aatacatttc agacgctgat 1080

ttgttggcta tagttagaga agttactgga aaattggtag aagagaaaat caaattagat 1140

gaattaactg ttgtatctgg aaataaaata acaccaattg catctgttaa actccattat 1200

aaaggagaag atataacttt aatagaaact gcttatggtg ttggaccggt agatgcagca 1260

ataaatgctg tgagaaaggc aataagtgga gttgcagata ttaagttggt agagtataga 1320

gttgaagcaa ttggtggagg aactgatgcg ttaatagagg ttgttgttaa attaagaaaa 1380

ggaactgaaa ttgttgaagt tagaaaatca gacgctgata taataagggc ttctgtagat 1440

gctgtaatgg aaggaatcaa tatgttattg aattaa 1476

<210> 3
<211> 491
<212> PRT
<213> Artificial Sequence

<220>
<223> CimA(M)m1

<400> 3
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Val Thr
        35                  40                  45

Ser Lys Gly Glu Arg Glu Gly Ile Lys Leu Ile Thr Lys Glu Gly Leu
50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Glu Thr Ala Leu Lys Ala Val Glu Tyr Ala Lys Glu His Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Lys Asn Val Asn Leu Pro Val Ser Val His
            180                 185                 190

Cys His Asn Asp Phe Gly Met Ala Thr Ala Asn Ala Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Leu Lys Ile
225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu Val Glu Glu Lys Ile Lys Leu Asp Glu Leu Thr Val
    370                 375                 380

Val Ser Gly Asn Lys Ile Thr Pro Ile Ala Ser Val Lys Leu His Tyr
385                 390                 395                 400

Lys Gly Glu Asp Ile Arg Leu Ile Glu Thr Ala Tyr Gly Val Gly Pro
                405                 410                 415

Val Asp Ala Ala Ile Asn Ala Val Arg Lys Ala Ile Ser Gly Val Ala
            420                 425                 430

Asp Ile Lys Leu Val Glu Tyr Arg Val Glu Ala Ile Gly Gly Gly Thr
        435                 440                 445

Asp Ala Leu Ile Glu Val Val Val Lys Leu Arg Lys Gly Thr Glu Ile
    450                 455                 460

Val Glu Val Arg Lys Ser Asp Ala Asp Ile Ile Arg Ala Ser Val Asp
465                 470                 475                 480

Ala Val Met Glu Gly Ile Asn Met Leu Leu Asn
                485                 490

<210>    4
<211>    1476
<212>    DNA
<213>    Artificial Sequence

<220>
<223>    cimA(M)m1

<400>    4
atgatggtaa ggatatttga tacaacactt agagatggag agcaaacacc aggagtttct      60

ttaacaccaa atgataagtt agagatagca aaaaaattgg atgagcttgg agttgatgtt     120

atagaggcag gttcagctgt aacttcaaaa ggagagagag aaggaataaa attaataaca     180

aaagaaggtt taaatgcaga aatctgctca tttgttagag ctttacctgt agatattgat     240

gctgccttag aatgtgatgt agatagtgtc catttagtag tgccaacatc tccaatacac     300

atgaaatata agcttagaaa aacagaagat gaggttttag agacagcttt aaaggctgta     360

gagtatgcta aagaacatgg attgattgtt gagttatctg cagaggatgc aacaagaagt     420

gatgtaaatt tcttaataaa actatttaat gaaggggaaa aggttggagc agacagagtt     480

tgtgtttgtg acacagtagg agttttaact ccacaaaaga gtcaggaatt atttaaaaaa     540

ataactaaaa atgttaattt accggtctca gttcattgcc acaacgactt tggaatggct     600

actgctaatg cttgctcagc agttttaggt ggagctgttc agtgccacgt aacagttaat     660

ggtattggag agagagcagg aaatgcctca ttggaagagg ttgttgctgc tttaaaaata     720

ctctatggct atgatactaa gataaagatg gaaaagttat atgaggtttc aagaattgtc     780

tcaagattga tgaaacttcc tgttccacca aataaagcaa ttgttgggga caatgcattt     840

gctcatgaag caggaataca tgttgatgga ttaataaaaa atactgaaac ctatgagcca     900

ataaaaccag aaatggttgg gaatagaaga agaattattt tgggtaagca ttctggtaga 960

aaagctttaa aatacaaact tgatttgatg ggcataaacg ttagtgatga gcaattaaat 1020

aaaatatatg aaagagttaa agaatttggg gatttgggta aatacatttc agacgctgat 1080

ttgttggcta tagttagaga agttactgga aaattggtag aagagaaaat caaattagat 1140

gaattaactg ttgtatctgg aaataaaata acaccaattg catctgttaa actccattat 1200

aaaggagaag atataaggtt aatagaaact gcttatggtg ttggaccggt agatgcagca 1260

ataaatgctg tgagaaaggc aataagtgga gttgcagata ttaagttggt agagtataga 1320

gttgaagcaa ttggtggagg aactgatgcg ttaatagagg ttgttgttaa attaagaaaa 1380

ggaactgaaa ttgttgaagt tagaaaatca gacgctgata taataagggc ttctgtagat 1440

gctgtaatgg aaggaatcaa tatgttattg aattaa 1476

<210> 5
<211> 491
<212> PRT
<213> Artificial Sequence

<220>
<223> CimA(M)m2

<400> 5
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Ile Thr
        35                  40                  45

Ser Lys Gly Glu Gly Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Val Glu Thr Ala Leu Lys Ala Val Glu Tyr Ala Lys Glu Gln Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Ala Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Leu Lys Ile
225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Glu Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu Val Glu Glu Lys Ile Lys Leu Asp Glu Leu Thr Val
    370                 375                 380

Val Ser Gly Asn Lys Ile Thr Pro Ile Ala Ser Val Lys Leu His Tyr
385                 390                 395                 400

Lys Gly Glu Asp Ile Thr Leu Ile Glu Thr Ala Tyr Gly Val Gly Pro
                405                 410                 415

Val Asp Ala Ala Ile Asn Ala Val Arg Lys Ala Ile Ser Gly Val Ala
            420                 425                 430

Asp Ile Lys Leu Val Glu Tyr Arg Val Glu Ala Ile Gly Gly Gly Thr
        435                 440                 445

Asp Ala Leu Ile Glu Val Val Val Lys Leu Arg Lys Gly Thr Glu Ile
    450                 455                 460

Val Glu Val Arg Lys Ser Asp Ala Asp Ile Ile Glu Ala Ser Val Asp
465                 470                 475                 480

Ala Val Met Glu Gly Ile Asn Met Leu Leu Asn
                485                 490

<210>   6

<211>    1476
<212>    DNA
<213>    Artificial Sequence

<220>
<223>    cimA(M)m2


<400>    6
atgatggtaa ggatatttga tacaacactt agagatggag agcaaacacc aggagtttct      60

ttaacaccaa atgataagtt agagatagca aaaaaattgg atgagcttgg agttgatgtt     120

atagaggcag gttcagctat aacttcaaaa ggagagggag aaggaataaa attaataaca     180

aaagaaggtt taaatgcaga aatctgctca tttgttagag ctttacctgt agatattgat     240

gctgccttag aatgtgatgt agatagtgtc catttagtag tgccaacatc tccaatacac     300

atgaaatata agcttagaaa aacagaagat gaggttgtag agacagcttt aaaggctgta     360

gagtatgcta aagaacaagg attgattgtt gagttatctg cagaggatgc aacaagaagt     420

gatgtaaatt tcttaataaa actatttaat gaaggggaaa aggttggagc agacagagtt     480

tgtgtttgtg acacagtagg agttttaact ccacaaaaga gtcaggaatt atttaaaaaa     540

ataactgaaa atgttaattt accggtctca gttcattgcc acaacgactt tggaatggct     600

actgctaatg cttgctcagc agttttaggt ggagctgttc agtgccacgt aacagttaat     660

ggtattggag agagagcagg aaatgcctca ttggaagagg ttgttgctgc tttaaaaata     720

ctctatggct atgatactaa gataaagatg gaaaagttat atgaggtttc aagaattgtc     780

tcaagattga tggagcttcc tgttccacca aataaagcaa ttgttgggga caatgcattt     840

gctcatgaag caggaataca tgttgatgga ttaataaaaa atactgaaac ctatgagcca     900

ataaaaccag aaatggttgg gaatagaaga agaattattt tgggtaagca ttctggtaga     960

aaagctttaa aatacaaact tgatttgatg ggcataaacg ttagtgatga gcaattaaat    1020

aaaatatatg aaagagttaa agaatttggg gatttgggta aatacatttc agacgctgat    1080

ttgttggcta tagttagaga agttactgga aaattggtag aagagaaaat caaattagat    1140

gaattaactg ttgtatctgg aaataaaata acaccaattg catctgttaa actccattat    1200

aaaggagaag atataacttt aatagaaact gcttatggtg ttggaccggt agatgcagca    1260

ataaatgctg tgagaaaggc aataagtgga gttgcagata ttaagttggt agagtataga    1320

gttgaagcaa ttggtggagg aactgatgcg ttaatagagg ttgttgttaa attaagaaaa    1380

ggaactgaaa ttgttgaagt tagaaaatca gacgctgata taatagaggc ttctgtagat    1440

gctgtaatgg aaggaatcaa tatgttattg aattaa                              1476


<210>    7
<211>    372
<212>    PRT

<213>    Artificial Sequence

<220>
<223>    CimA(M)m3


<400>    7
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Val Thr
        35                  40                  45

Ser Lys Gly Glu Arg Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Val Thr Ala Leu Lys Ala Val Glu Tyr Ala Lys Glu Gln Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Ala Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Ser Lys Ile
225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu
    370

<210>    8
<211>    1119
<212>    DNA
<213>    Artificial Sequence

<220>
<223>    cimA(M)m3

<400>    8
atgatggtaa ggatattcga taccacactt cgcgacggag agcagacacc aggagtttct      60

ttaacaccaa acgataagct ggagatagct aaaaagttgg atgaacttgg ggttgatgtt     120

atagaggccg gtagcgctgt cactagcaag ggagagcgcg aaggaataaa actcattaca     180

aaagaaggtc ttaacgcaga aatctgcagt ttcgtgcgtg cactccctgt agacatagac     240

gctgccttag aatgtgatgt agatagtgtg cacctcgtgg tgcccacatc tccaatacac     300

atgaagtaca aacttcgtaa aacggaggat gaggttctcg tgaccgcact taaggctgtg     360

gaatacgcta aagagcaggg cttgatcgtt gagttatcag cggaggatgc gacaagatcc     420

gatgttaatt tcttaataaa actcttcaat gaaggggaaa aggttggagc agaccgtgtt     480

tgtgtttgcg acaccgtagg agttttaacg ccacaaaagt cgcaggaact gtttaagaaa     540

ataaccgaga atgtgaattt accggtctcg gttcattgcc acaacgactt tggaatggct     600

actgctaatg cttgctccgc agtgctcggc ggagctgttc agtgccacgt gacagttaac     660

ggtattggcg agcgcgcagg gaatgcctcc ttagaagagg tcgttgccgc gtcaaaaatc     720

ctctacggct atgatactaa aataaagatg gaaaagttat acgaggtttc acgcattgtc     780

tcccgcttga tgaaacttcc tgttcccccg aacaaagcga ttgttggcga caatgcgttc     840

gctcacgaag caggtatcca tgtcgatgga ttaattaaaa atactgaaac ctatgagcca     900

atcaaaccag aaatggtggg caaccgccgg cgtatcattc tcggtaagca ctctggtcgg     960

aaagcgttaa agtacaagct tgatctaatg ggcataaacg tgtctgatga gcaattaaac    1020

aaaatttatg aacgcgttaa agaattcggg gacctaggta agtacatttc agatgctgat    1080

ttgctcgcta tagttcgcga agtcacggga aagttgtaa                                1119

<210>    9
<211>    372
<212>    PRT
<213>    Artificial Sequence

<220>
<223>    CimA(M)m4

<400>    9
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Val Thr
        35                  40                  45

Ser Lys Gly Glu Arg Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Glu Thr Ala Leu Lys Ala Val Glu Tyr Ala Lys Glu Gln Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Thr Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Leu Lys Ile
225                 230                 235                 240

Leu Tyr Gly Tyr Asp Thr Lys Ile Lys Met Glu Lys Ser Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu
    370

<210>   10
<211>   1119
<212>   DNA
<213>   Artificial Sequence

<220>
<223>   cimA(M)m4

<400>   10
atgatggtaa ggatatttga tacaacactt agagatggag agcaaacacc aggagtttct      60

ttaacaccaa atgataagtt agagatagca aaaaaattgg atgagcttgg agttgatgtt     120

atagaggcag gttcagctgt aacttcaaaa ggagagagag aaggaataaa attaataaca     180

aaagaaggtt taaatgcaga aatctgctca tttgttagag ctttacctgt agatattgat     240

gctgccttag aatgtgatgt agatagtgtc catttagtag tgccaacatc tccaatacac     300

atgaaatata agcttagaaa aacagaagat gaggttttag agacagcttt aaaggctgta     360

gagtatgcta aagaacaagg attgattgtt gagttatctg cagaggatgc aacaagaagt     420

gatgtaaatt tcttaataaa actatttaat gaaggggaaa aggttggagc agacagagtt     480

tgtgtttgtg acacagtagg agttttaact ccacaaaaga gtcaggaatt atttaaaaaa     540

ataactgaaa atgttaattt accggtctca gttcattgcc acaacgactt tggaatggct     600

actgctaata cttgctcagc agttttaggt ggagctgttc agtgccacgt aacagttaat     660

ggtattggag agagagcagg aaatgcctca ttggaagagg ttgttgctgc tttaaaaata     720

ctctatggct atgatactaa gataaagatg gaaaagtcat atgaggtttc aagaattgtc     780

tcaagattga tgaaacttcc tgttccacca aataaagcaa ttgttgggga caatgcattt     840

gctcatgaag caggaataca tgttgatgga ttaataaaaa atactgaaac ctatgagcca     900

ataaaaccag aaatggttgg gaatagaaga agaattattt tgggtaagca ttctggtaga 960

aaagctttaa aatacaaact tgatttgatg ggcataaacg ttagtgatga gcaattaaat 1020

aaaatatatg aaagagttaa agaatttggg gatttgggta aatacatttc agacgctgat 1080

ttgttggcta tagttagaga agttactgga aaattgtga 1119

<210> 11
<211> 491
<212> PRT
<213> Artificial Sequence

<220>
<223> CimA(M)m5

<400> 11
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Ile Thr
        35                  40                  45

Ser Lys Gly Glu Arg Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Glu Thr Ala Leu Lys Ala Val Glu Tyr Ala Lys Glu His Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Ala Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Ser Lys Ile
225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu Val Glu Glu Lys Ile Lys Leu Asp Glu Leu Thr Val
    370                 375                 380

Val Ser Gly Asn Lys Ile Thr Pro Ile Ala Ser Val Lys Leu His Tyr
385                 390                 395                 400

Lys Gly Glu Asp Ile Thr Leu Ile Glu Thr Ala Tyr Gly Val Gly Pro
                405                 410                 415

Val Asp Ala Ala Ile Asn Ala Val Arg Lys Ala Ile Ser Gly Val Ala
            420                 425                 430

Asp Ile Lys Leu Val Glu Tyr Arg Ala Glu Ala Ile Gly Gly Gly Thr
        435                 440                 445

Asp Ala Leu Ile Glu Val Val Val Lys Leu Arg Lys Gly Thr Glu Ile
    450                 455                 460

Val Glu Val Arg Lys Ser Asp Ala Asp Ile Ile Arg Ala Ser Val Asp
465                 470                 475                 480

Ala Val Met Glu Gly Ile Asn Met Leu Leu Asn
                485                 490


<210>    12
<211>    1476
<212>    DNA
<213>    Artificial Sequence

<220>
<223>    cimA(M)m5


<400>    12
atgatggtaa ggatatttga tacaacactt agagatggag agcaaacacc aggagtttct        60

ttaacaccaa atgataagtt agagatagca aaaaaattgg atgagcttgg agttgatgtt       120

atagaggcag gttcagctat aacttcaaaa ggagagagag aaggaataaa attaataaca 180

aaagaaggtt taaatgcaga aatctgctca tttgttagag ctttacctgt agatattgat 240

gctgccttag aatgtgatgt agatagtgtc catttagtag tgccaacatc tccaatacac 300

atgaaatata agcttagaaa aacagaagat gaggttttag agacagcttt aaaggctgta 360

gagtatgcta aagaacatgg attgattgtt gagttatctg cagaggatgc aacaagaagt 420

gatgtaaatt tcttaataaa actatttaat gaaggggaaa aggttggagc agacagagtt 480

tgtgtttgtg acacagtagg agttttaact ccacaaaaga gtcaggaatt atttaaaaaa 540

ataactgaaa atgttaattt accggtctca gttcattgcc acaacgactt tggaatggct 600

actgctaatg cttgctcagc agttttaggt ggagctgttc agtgccacgt aacagttaat 660

ggtattggag agagagcagg aaatgcctca ttggaagagg ttgttgctgc ttcaaaaata 720

ctctatggct atgatactaa gataaagatg gaaaagttat atgaggtttc aagaattgtc 780

tcaagattga tgaaacttcc tgttccacca aataaagcaa ttgttgggga caatgcattt 840

gctcatgaag caggaataca tgttgatgga ttaataaaaa atactgaaac ctatgagcca 900

ataaaaccag aaatggttgg gaatagaaga agaattattt tgggtaagca ttctggtaga 960

aaagctttaa aatacaaact tgatttgatg ggcataaacg ttagtgatga gcaattaaat 1020

aaaatatatg aaagagttaa agaatttggg gatttgggta aatacatttc agacgctgat 1080

ttgttggcta tagttagaga agttactgga aaattggtag aagagaaaat caaattagat 1140

gaattaactg ttgtatctgg aaataaaata acaccaattg catctgttaa actccattat 1200

aaaggagaag atataacttt aatagaaact gcttatggtg ttggaccggt agatgcagca 1260

ataaatgctg tgagaaaggc aataagtgga gttgcagata ttaagttggt agagtataga 1320

gccgaagcaa ttggtggagg aactgatgcg ttaatagagg ttgttgttaa attaagaaaa 1380

ggaactgaaa ttgttgaagt tagaaaatca gacgctgata taataagggc ttctgtagat 1440

gctgtaatgg aaggaatcaa tatgttattg aattaa 1476

<210> 13
<211> 491
<212> PRT
<213> Artificial Sequence

<220>
<223> CimA(M)m6

<400> 13
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Val Thr
        35                  40                  45

Ser Lys Gly Glu Arg Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Glu Thr Ala Leu Lys Ala Val Glu Tyr Ala Lys Glu Gln Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Ala Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Leu Lys Ile
225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Lys
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu Val Glu Glu Lys Ile Lys Leu Asp Glu Leu Thr Val
    370                 375                 380

Val Ser Gly Asn Lys Ile Thr Pro Ile Ala Ser Val Lys Leu His Tyr
385                 390                 395                 400

Lys Gly Glu Asp Ile Thr Leu Ile Glu Thr Ala Tyr Gly Val Gly Pro
                405                 410                 415

Val Asp Ala Ala Ile Asn Ala Val Arg Lys Ala Ile Ser Gly Val Ala
            420                 425                 430

Asp Ile Asn Leu Val Glu Tyr Arg Val Glu Ala Ile Gly Gly Gly Thr
        435                 440                 445

Asp Ala Leu Ile Glu Val Val Val Lys Leu Arg Lys Gly Thr Glu Ile
    450                 455                 460

Val Glu Val Arg Lys Ser Asp Ala Asp Ile Ile Arg Ala Ser Val Asp
465                 470                 475                 480

Ala Val Met Glu Gly Ile Asn Met Leu Leu Asn
                485                 490


<210>    14
<211>    1476
<212>    DNA
<213>    Artificial Sequence

<220>
<223>    cimA(M)m6


<400>    14
atgatggtaa ggatatttga tacaacactt agagatggag agcaaacacc aggagtttct      60

ttaacaccaa atgataagtt agagatagca aaaaaattgg atgagcttgg agttgatgtt     120

atagaggcag gttcagctgt aacttcaaaa ggagagagag aaggaataaa attaataaca     180

aaagaaggtt taaatgcaga aatctgctca tttgttagag ctttacctgt agatattgat     240

gctgccttag aatgtgatgt agatagtgtc catttagtag tgccaacatc tccaatacac     300

atgaaatata agcttagaaa aacagaagat gaggttttag agacagcttt aaaggctgta     360

gagtatgcta aagaacaagg attgattgtt gagttatctg cagaggatgc aacaagaagt     420

gatgtaaatt tcttaataaa actatttaat gaaggggaaa aggttggagc agacagagtt     480

tgtgtttgtg acacagtagg agttttaact ccacaaaaga gtcaggaatt atttaaaaaa     540

ataactgaaa atgttaattt accggtctca gttcattgcc acaacgactt tggaatggct     600

actgctaatg cttgctcagc agttttaggt ggagctgttc agtgccacgt aacagttaat     660

ggtattggag agagagcagg aaatgcctca ttggaagagg ttgttgctgc tttaaaaata     720

ctctatggct atgatactaa gataaagatg gaaaagttat atgaggtttc aagaattgtc     780

tcaagattga tgaaacttcc tgttccacca aataaagcaa ttgttgggga caatgcattt     840

gctcatgaag caggaataca tgttgatgga ttaataaaaa atactgaaac ctatgagcca 900

ataaaaccag aaatggttgg gaatagaaga agaattattt tgggtaagca ttctggtaga 960

aaagctttaa aatacaaact tgatttgatg ggcataaacg ttagtaaaga gcaattaaat 1020

aaaatatatg aaagagttaa agaatttggg gatttgggta aatacatttc agacgctgat 1080

ttgttggcta tagttagaga agttactgga aaattggtag aagagaaaat caaattagat 1140

gaattaactg ttgtatctgg aaataaaata acaccaattg catctgttaa actccattat 1200

aaaggagaag atataacttt aatagaaact gcttatggtg ttggaccggt agatgcagca 1260

ataaatgctg tgagaaaggc aataagtgga gttgcagata ttaacttggt agagtataga 1320

gttgaagcaa ttggtggagg aactgatgcg ttaatagagg ttgttgttaa attaagaaaa 1380

ggaactgaaa ttgttgaagt tagaaaatca gacgctgata taataagggc ttctgtagat 1440

gctgtaatgg aaggaatcaa tatgttattg aattaa 1476

<210> 15
<211> 491
<212> PRT
<213> Artificial Sequence

<220>
<223> CimA(M)m7

<400> 15
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Val Thr
        35                  40                  45

Ser Lys Gly Glu Arg Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Met Thr Ala Leu Lys Ala Val Glu Tyr Ala Lys Glu His Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Thr Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Leu Lys Ile
225                 230                 235                 240

Leu Tyr Gly Tyr Asp Thr Lys Ile Lys Gln Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu Val Glu Glu Lys Ile Lys Leu Asp Glu Leu Thr Val
    370                 375                 380

Val Ser Gly Asn Lys Ile Thr Pro Ile Ala Ser Val Lys Leu His Tyr
385                 390                 395                 400

Lys Gly Glu Asp Ile Thr Leu Ile Glu Thr Ala Tyr Gly Val Gly Pro
                405                 410                 415

Val Asp Ala Ala Ile Asn Ala Val Arg Lys Ala Ile Ser Gly Val Ala
            420                 425                 430

Asp Ile Lys Leu Val Glu Tyr Arg Ala Glu Ala Ile Gly Gly Gly Thr
        435                 440                 445

Asp Ala Leu Ile Glu Val Val Val Lys Leu Arg Lys Gly Thr Glu Ile
    450                 455                 460

Val Glu Val Arg Lys Ser Asp Ala Asp Ile Ile Arg Ala Ser Val Asp
465                 470                 475                 480

Ala Val Met Glu Gly Ile Asn Met Leu Leu Asn
                485                 490

<210>    16
<211>    1476
<212>    DNA
<213>    Artificial Sequence

<220>
<223>    cimA(M)m7


<400>    16
atgatggtaa ggatatttga tacaacactt agagatggag agcaaacacc aggagtttct      60

ttaacaccaa atgataagtt agagatagca aaaaaattgg atgagcttgg agttgatgtt     120

atagaggcag gttcagctgt aacttcaaaa ggagagagag aaggaataaa attaataaca     180

aaagaaggtt taaatgcaga aatctgctca tttgttagag ctttacctgt agatattgat     240

gctgccttag aatgtgatgt agatagtgtc catttagtag tgccaacatc tccaatacac     300

atgaaatata agcttagaaa aacagaagat gaggttttaa tgacagcttt aaaggctgta     360

gagtatgcta aagaacatgg attgattgtt gagttatctg cagaggatgc aacaagaagt     420

gatgtaaatt tcttaataaa actatttaat gaaggggaaa aggttggagc agacagagtt     480

tgtgtttgtg acacagtagg agttttaact ccacaaaaga gtcaggaatt atttaaaaaa     540

ataactgaaa atgttaattt accggtctca gttcattgcc acaacgactt tggaatggct     600

actgctaata cttgctcagc agttttaggt ggagctgttc agtgccacgt aacagttaat     660

ggtattggag agagagcagg aaatgcctca ttggaagagg ttgttgctgc tttaaaaata     720

ctctatggct atgatactaa gataaagcag gaaaagttat atgaggtttc aagaattgtc     780

tcaagattga tgaaacttcc tgttccacca aataaagcaa ttgttgggga caatgcattt     840

gctcatgaag caggaataca tgttgatgga ttaataaaaa atactgaaac ctatgagcca     900

ataaaaccag aaatggttgg gaatagaaga agaattattt tgggtaagca ttctggtaga     960

aaagctttaa aatacaaact tgatttgatg ggcataaacg ttagtgatga gcaattaaat    1020

aaaatatatg aaagagttaa agaatttggg gatttgggta aatacatttc agacgctgat    1080

ttgttggcta tagttagaga agttactgga aaattggtag aagagaaaat caaattagat    1140

gaattaactg ttgtatctgg aaataaaata acaccaattg catctgttaa actccattat    1200

aaaggagaag atataacttt aatagaaact gcttatggtg ttggaccggt agatgcagca    1260

ataaatgctg tgagaaaggc aataagtgga gttgcagata ttaagttggt agagtataga    1320

gctgaagcaa ttggtggagg aactgatgcg ttaatagagg ttgttgttaa attaagaaaa    1380

ggaactgaaa ttgttgaagt tagaaaatca gacgctgata taataagggc ttctgtagat    1440

gctgtaatgg aaggaatcaa tatgttattg aattaa                              1476

<210>    17
<211>    340
<212>    PRT
<213>    leuB from Corynebacterium glutamicum ATCC13032

<400>    17
Met Lys Leu Ala Val Ile Gly Gly Asp Gly Ile Gly Pro Glu Val Thr
1               5                   10                  15

Ala Glu Ala Leu Lys Val Leu Asn Ala Val Arg Asp Asp Ile Glu Thr
            20                  25                  30

Thr Asp Tyr Asp Leu Gly Ala Arg Arg Tyr Leu Lys Asn Gly Glu Leu
        35                  40                  45

Leu Thr Asp Glu Asp Leu Ala Ser Leu Arg Glu His Asp Ala Ile Leu
    50                  55                  60

Leu Gly Ala Ile Gly Ala Pro Gly Ser Val Pro Pro Gly Ile Leu Glu
65                  70                  75                  80

Arg Gly Leu Leu Leu Lys Met Arg Phe Ala Leu Asp His His Val Asn
                85                  90                  95

Leu Arg Pro Ser Lys Leu Tyr Asp Gly Val Glu Ser Pro Leu Arg Asn
            100                 105                 110

Pro Gly Lys Ile Asp Phe Val Val Val Arg Glu Gly Thr Glu Gly Ala
        115                 120                 125

Tyr Thr Gly Asn Gly Gly Ala Ile Arg Val Gly Thr Pro His Glu Ile
    130                 135                 140

Ala Asn Glu Thr Ser Val Asn Thr Arg Tyr Gly Ala Glu Arg Val Ile
145                 150                 155                 160

Arg Tyr Ala Phe Glu Leu Ala Gln Ser Arg Arg Lys Lys Leu Thr Leu
                165                 170                 175

Val His Lys Thr Asn Val Leu Val His Gly Gly Gly Leu Trp Gln Arg
            180                 185                 190

Thr Val Asp Glu Val Ala Lys Glu Tyr Pro Glu Val Ala Val Asp Tyr
        195                 200                 205

Asn His Ile Asp Ala Ala Thr Ile Tyr Leu Val Thr Asp Pro Ser Arg
    210                 215                 220

Phe Asp Val Ile Val Thr Asp Asn Leu Phe Gly Asp Ile Leu Thr Asp
225                 230                 235                 240

Glu Ala Gly Ala Val Ser Gly Gly Ile Gly Leu Ala Ala Ser Gly Asn
                245                 250                 255

Ile Asp Ala Thr Gly Thr Asn Pro Ser Met Phe Glu Pro Val His Gly
            260                 265                 270

Ser Ala Pro Asp Ile Ala Gly Gln Gly Ile Ala Asp Pro Thr Ala Ala
        275                 280                 285

Ile Leu Ser Ala Ala Met Leu Leu Arg His Leu Gly Asp Glu Asp Asn
    290                 295                 300

Ala Val Arg Ile Glu Thr Ala Ile Ala Ala Asp Val Ala Gly Arg Asp
305                 310                 315                 320

Asn Ser Gln Pro Ile Ser Thr Thr Glu Val Gly Asp Arg Ile Val Lys
                325                 330                 335

Ala Leu Gln Ser
            340

<210>    18
<211>    1023
<212>    DNA
<213>    leuB from Corynebacterium glutamicum ATCC13032

<400>    18
atgaaacttg ctgttattgg tggagatggt atcggcccag aggttactgc agaagccctc      60

aaggttctaa acgctgtccg cgacgacatc gagaccaccg attatgacct tggcgcacgc     120

cgttacctca aaaatggcga gctgctcacc gacgaggatc tggcatccct gcgcgagcat     180

gacgcgatcc ttcttggcgc tatcggtgca ccaggttccg tccctccagg aattctcgag     240

cgtggtttgc tgctgaagat gcgattcgca ctggatcacc acgtgaacct gcgcccatcc     300

aagctgtacg acggcgtgga gtccccactg cgtaacccag gcaagattga tttcgttgtg     360

gtccgcgaag gtaccgaagg cgcctacact ggcaacggtg gagcaatccg cgtgggaacc     420

cctcacgaga ttgccaatga aacctccgtg aacactcgct acggcgctga gcgcgttatt     480

cgctacgcat tcgagctggc acagagccgc cgcaagaagc tcaccctcgt gcacaagacc     540

aacgtcctgg ttcacggtgg tggcctgtgg cagcgcaccg tagatgaggt tgcaaaggaa     600

tacccagagg tagccgtcga ttacaaccac atcgatgcag caaccatcta tctggtcact     660

gatccttccc gcttcgatgt gattgttacc gataacctct tcggcgacat cctcaccgat     720

gaggcaggcg cagtctctgg cggaattggc ctcgcagcat ccggcaacat cgatgccacg     780

ggcaccaacc cttccatgtt cgagccagtc cacggctctg caccagatat cgcaggccag     840

ggaatcgcag acccaacggc agcaatccta tccgctgcga tgctgctgcg tcacttaggt     900

gatgaagaca acgcagtacg tattgaaaca gccatcgcag ctgatgtggc tggccgagat     960

aactctcagc cgatttctac cactgaggtg ggagaccgca tcgtcaaggc gctgcaaagc    1020

taa                                                                  1023

<210>    19
<211>    481
<212>    PRT
<213>    leuC from Corynebacterium glutamicum ATCC13032

<400>    19
Met Thr Ser Pro Val Glu Asn Ser Thr Ser Thr Glu Lys Leu Thr Leu
  1               5                  10                  15

Ala Glu Lys Val Trp Arg Asp His Val Val Ser Lys Gly Glu Asn Gly

20                  25                  30

Glu Pro Asp Leu Leu Tyr Ile Asp Leu Gln Leu Leu His Glu Val Thr
        35                  40                  45

Ser Pro Gln Ala Phe Asp Gly Leu Arg Met Thr Gly Arg Lys Leu Arg
    50                  55                  60

His Pro Glu Leu His Leu Ala Thr Glu Asp His Asn Val Pro Thr Glu
65                  70                  75                  80

Gly Ile Lys Thr Gly Ser Leu Leu Glu Ile Asn Asp Lys Ile Ser Arg
                85                  90                  95

Leu Gln Val Ser Thr Leu Arg Asp Asn Cys Glu Glu Phe Gly Val Arg
            100                 105                 110

Leu His Pro Met Gly Asp Val Arg Gln Gly Ile Val His Thr Val Gly
        115                 120                 125

Pro Gln Leu Gly Ala Thr Gln Pro Gly Met Thr Ile Val Cys Gly Asp
    130                 135                 140

Ser His Thr Ser Thr His Gly Ala Phe Gly Ser Met Ala Phe Gly Ile
145                 150                 155                 160

Gly Thr Ser Glu Val Glu His Val Met Ala Thr Gln Thr Leu Pro Leu
                165                 170                 175

Lys Pro Phe Lys Thr Met Ala Ile Glu Val Thr Gly Glu Leu Gln Pro
            180                 185                 190

Gly Val Ser Ser Lys Asp Leu Ile Leu Ala Ile Ile Ala Lys Ile Gly
        195                 200                 205

Thr Gly Gly Gly Gln Gly Tyr Val Leu Glu Tyr Arg Gly Glu Ala Ile
    210                 215                 220

Arg Lys Met Ser Met Asp Ala Arg Met Thr Met Cys Asn Met Ser Ile
225                 230                 235                 240

Glu Ala Gly Ala Arg Ala Gly Met Ile Ala Pro Asp Gln Thr Thr Phe
                245                 250                 255

Asp Tyr Val Glu Gly Arg Glu Met Ala Pro Lys Gly Ala Asp Trp Asp
            260                 265                 270

Glu Ala Val Ala Tyr Trp Lys Thr Leu Pro Thr Asp Glu Gly Ala Thr
        275                 280                 285

Phe Asp Lys Val Val Glu Ile Asp Gly Ser Ala Leu Thr Pro Phe Ile
    290                 295                 300

Thr Trp Gly Thr Asn Pro Gly Gln Gly Leu Pro Leu Gly Glu Ser Val
305                 310                 315                 320

Pro Ser Pro Glu Asp Phe Thr Asn Asp Asn Asp Lys Ala Ala Ala Glu
                325                 330                 335

Lys Ala Leu Gln Tyr Met Asp Leu Val Pro Gly Thr Pro Leu Arg Asp
            340                 345                 350

Ile Lys Ile Asp Thr Val Phe Leu Gly Ser Cys Thr Asn Ala Arg Ile

355                 360                 365

Glu Asp Leu Gln Ile Ala Ala Asp Ile Leu Lys Gly His Lys Ile Ala
    370                 375                 380

Asp Gly Met Arg Met Met Val Val Pro Ser Ser Thr Trp Ile Lys Gln
385                 390                 395                 400

Glu Ala Glu Ala Leu Gly Leu Asp Lys Ile Phe Thr Asp Ala Gly Ala
                405                 410                 415

Glu Trp Arg Thr Ala Gly Cys Ser Met Cys Leu Gly Met Asn Pro Asp
            420                 425                 430

Gln Leu Lys Pro Gly Glu Arg Ser Ala Ser Thr Ser Asn Arg Asn Phe
        435                 440                 445

Glu Gly Arg Gln Gly Pro Gly Gly Arg Thr His Leu Val Ser Pro Ala
    450                 455                 460

Val Ala Ala Ala Thr Ala Ile Arg Gly Thr Leu Ser Ser Pro Ala Asp
465                 470                 475                 480

Ile

<210>    20
<211>    1446
<212>    DNA
<213>    leuC from Corynebacterium glutamicum ATCC13032

<400>    20
atgaccagcc ccgtggagaa cagcacctca actgagaagc tgaccctggc agagaaggtg        60

tggcgcgacc atgtcgtgtc caagggagaa aacggcgagc ccgacctcct ctacatcgac       120

ctgcagctgc tgcatgaagt gacctcacca caggcatttg acggcctgcg catgaccggc       180

cgtaaactgc gccacccaga actgcacctg gccaccgaag accacaacgt gccaaccgaa       240

ggcatcaaga ctggctcact gctggaaatc aacgacaaga tttcccgcct gcaggtatct       300

actctgcgcg acaactgtga agaattcggc gtgcgcctgc acccaatggg tgatgtccga       360

cagggcatcg tgcacaccgt cggcccacag ctcggcgcaa cccagccagg catgaccatt       420

gtgtgcggtg actcccacac ctccacccac ggtgcttttg gctccatggc attcggcatc       480

ggtacctcag aggttgagca cgtcatggct actcaaaccc tgccactgaa gcctttcaag       540

accatggcca ttgaagttac tggtgaactg cagccaggtg tttcctccaa ggacctgatt       600

ctggcgatta tcgccaagat cggcaccggc ggcggacagg gctacgttct ggaataccgc       660

ggcgaagcaa tccgtaagat gtccatggat gcacgcatga ccatgtgcaa catgtccatc       720

gaagctggcg cacgtgccgg catgatcgcc ccagaccaaa ccaccttcga ctacgttgaa       780

ggccgcgaaa tggcaccaaa gggcgccgac tgggacgaag cagttgctta ctggaagacc       840

ctgccaaccg acgaaggcgc aacctttgac aaggtcgtag aaatcgatgg ctccgcactg       900

accccattca tcacctgggg caccaaccca ggccagggcc tgccactggg cgaatccgta 960

ccaagcccag aagacttcac caacgacaac gacaaggcag cagccgaaaa ggcactgcag 1020

tacatggacc tggtaccagg aaccccactg cgcgacatca agatcgacac cgtcttcctg 1080

ggatcctgca ccaacgcccg catcgaagac ctgcagatcg ccgctgacat cctcaagggc 1140

cacaaaatcg ccgacggcat gcgcatgatg gtcgtgcctt cctccacctg gatcaagcaa 1200

gaggcagaag cgctcggact ggacaaaatc ttcaccgacg ctggcgctga atggcgtacc 1260

gcaggctgct ccatgtgcct gggcatgaac ccagaccaac tgaagccagg cgagcgctcc 1320

gcatccacct ccaaccgaaa cttcgaagga cgccaaggac caggaggccg cacccacctg 1380

gtatccccag cagtcgcagc cgccaccgca atccgcggca ccctgtcctc acctgcagat 1440

atctaa 1446

<210> 21
<211> 197
<212> PRT
<213> leuD from Corynebacterium glutamicum ATCC13032

<400> 21
Met Glu Lys Phe Thr Thr Tyr Thr Gly Val Gly Val Pro Leu Gln Arg
1               5                   10                  15

Ser Asn Val Asp Thr Asp Gln Ile Ile Pro Ala Val Tyr Leu Lys Arg
            20                  25                  30

Val Thr Arg Thr Gly Phe Glu Asp Gly Leu Phe Ser Asn Trp Arg Gln
        35                  40                  45

Asn Asp Pro Asn Phe Val Leu Asn Thr Asp Thr Tyr Lys Asn Gly Ser
    50                  55                  60

Val Leu Val Ala Gly Pro Asp Phe Gly Thr Gly Ser Ser Arg Glu His
65                  70                  75                  80

Ala Val Trp Ala Leu Met Asp Tyr Gly Phe Arg Ala Val Phe Ser Ser
                85                  90                  95

Arg Phe Ala Asp Ile Phe Arg Gly Asn Ser Gly Lys Ala Gly Met Leu
            100                 105                 110

Thr Gly Ile Met Glu Gln Ser Asp Ile Glu Leu Leu Trp Lys Leu Met
        115                 120                 125

Glu Gln Thr Pro Gly Leu Glu Leu Thr Val Asn Leu Glu Lys Gln Ile
    130                 135                 140

Val Thr Ala Gly Asp Val Val Ile Ser Phe Glu Val Asp Pro Tyr Ile
145                 150                 155                 160

Arg Trp Arg Leu Met Glu Gly Leu Asp Asp Ala Gly Leu Thr Leu Arg
                165                 170                 175

Lys Leu Asp Glu Ile Glu Asp Tyr Glu Ala Lys Arg Pro Ala Phe Lys
            180                 185                 190

Pro Arg Thr Asn Ala
        195

<210>    22
<211>    594
<212>    DNA
<213>    leuD from Corynebacterium glutamicum ATCC13032

<400>    22
atggaaaaat ttaccaccta caccggcgtt ggcgttccac tgcagcgatc caacgtggac        60

accgaccaga tcatcccagc cgtctacctc aagcgcgtca cccggaccgg cttcgaagac       120

ggactgtttt ccaactggcg ccaaaacgac cccaactttg tcctcaacac cgacacctac       180

aagaacggct ccgttctcgt agcaggccct gactttggca ccggctcctc ccgcgagcac       240

gccgtctggg cactcatgga ctacggcttc cgcgctgtct tctcctcacg attcgccgac       300

atcttccgcg gcaactccgg aaaagcgggc atgctcaccg gcatcatgga acagtccgac       360

atcgaacttc tgtggaagct catggaacaa accccaggcc tcgaactgac cgtgaacctg       420

gaaaagcaga tcgtcaccgc aggcgacgta gtgatcagct tcgaagttga cccctacatc       480

cgctggcgtt tgatggaagg cctcgacgac gctggcctga ccctgcgcaa gctcgatgaa       540

attgaagact acgaggctaa gcgccctgcg tttaagccac gcactaacgc ttaa             594

<210>    23
<211>    436
<212>    PRT
<213>    ilvA from Corynebacterium glutamicum ATCC13032

<400>    23
Met Ser Glu Thr Tyr Val Ser Glu Lys Ser Pro Gly Val Met Ala Ser
 1               5                   10                  15

Gly Ala Glu Leu Ile Arg Ala Ala Asp Ile Gln Thr Ala Gln Ala Arg
            20                  25                  30

Ile Ser Ser Val Ile Ala Pro Thr Pro Leu Gln Tyr Cys Pro Arg Leu
        35                  40                  45

Ser Glu Glu Thr Gly Ala Glu Ile Tyr Leu Lys Arg Glu Asp Leu Gln
    50                  55                  60

Asp Val Arg Ser Tyr Lys Ile Arg Gly Ala Leu Asn Ser Gly Ala Gln
65                  70                  75                  80

Leu Thr Gln Glu Gln Arg Asp Ala Gly Ile Val Ala Ala Ser Ala Gly
                85                  90                  95

Asn His Ala Gln Gly Val Ala Tyr Val Cys Lys Ser Leu Gly Val Gln
            100                 105                 110

Gly Arg Ile Tyr Val Pro Val Gln Thr Pro Lys Gln Lys Arg Asp Arg
        115                 120                 125

Ile Met Val His Gly Gly Glu Phe Val Ser Leu Val Val Thr Gly Asn
    130                 135                 140

Asn Phe Asp Glu Ala Ser Ala Ala Ala His Glu Asp Ala Glu Arg Thr
145                 150                 155                 160

Gly Ala Thr Leu Ile Glu Pro Phe Asp Ala Arg Asn Thr Val Ile Gly
                165                 170                 175

Gln Gly Thr Val Ala Ala Glu Ile Leu Ser Gln Leu Thr Ser Met Gly
            180                 185                 190

Lys Ser Ala Asp His Val Met Val Pro Val Gly Gly Gly Gly Leu Leu
        195                 200                 205

Ala Gly Val Val Ser Tyr Met Ala Asp Met Ala Pro Arg Thr Ala Ile
    210                 215                 220

Val Gly Ile Glu Pro Ala Gly Ala Ala Ser Met Gln Ala Ala Leu His
225                 230                 235                 240

Asn Gly Gly Pro Ile Thr Leu Glu Thr Val Asp Pro Phe Val Asp Gly
                245                 250                 255

Ala Ala Val Lys Arg Val Gly Asp Leu Asn Tyr Thr Ile Val Glu Lys
            260                 265                 270

Asn Gln Gly Arg Val His Met Met Ser Ala Thr Glu Gly Ala Val Cys
        275                 280                 285

Thr Glu Met Leu Asp Leu Tyr Gln Asn Glu Gly Ile Ile Ala Glu Pro
    290                 295                 300

Ala Gly Ala Leu Ser Ile Ala Gly Leu Lys Glu Met Ser Phe Ala Pro
305                 310                 315                 320

Gly Ser Val Val Val Cys Ile Ile Ser Gly Gly Asn Asn Asp Val Leu
                325                 330                 335

Arg Tyr Ala Glu Ile Ala Glu Arg Ser Leu Val His Arg Gly Leu Lys
            340                 345                 350

His Tyr Phe Leu Val Asn Phe Pro Gln Lys Pro Gly Gln Leu Arg His
        355                 360                 365

Phe Leu Glu Asp Ile Leu Gly Pro Asp Asp Asp Ile Thr Leu Phe Glu
    370                 375                 380

Tyr Leu Lys Arg Asn Asn Arg Glu Thr Gly Thr Ala Leu Val Gly Ile
385                 390                 395                 400

His Leu Ser Glu Ala Ser Gly Leu Asp Ser Leu Leu Glu Arg Met Glu
                405                 410                 415

Glu Ser Ala Ile Asp Ser Arg Arg Leu Glu Pro Gly Thr Pro Glu Tyr
            420                 425                 430

Glu Tyr Leu Thr
        435


<210>    24
<211>    1311
<212>    DNA
<213>    ilvA from Corynebacterium glutamicum ATCC13032

<400>    24
atgagtgaaa catacgtgtc tgagaaaagt ccaggagtga tggctagcgg agcggagctg      60

attcgtgccg ccgacattca aacggcgcag gcacgaattt cctccgtcat tgcaccaact     120

ccattgcagt attgccctcg tctttctgag gaaaccggag cggaaatcta ccttaagcgt     180

gaggatctgc aggatgttcg ttcctacaag atccgcggtg cgctgaactc tggagcgcag     240

ctcacccaag agcagcgcga tgcaggtatc gttgccgcat ctgcaggtaa ccatgcccag     300

ggcgtggcct atgtgtgcaa gtccttgggc gttcagggac gcatctatgt tcctgtgcag     360

actccaaagc aaaagcgtga ccgcatcatg gttcacggcg gagagtttgt ctccttggtg     420

gtcactggca ataacttcga cgaagcatcg gctgcagcgc atgaagatgc agagcgcacc     480

ggcgcaacgc tgatcgagcc tttcgatgct cgcaacaccg tcatcggtca gggcaccgtg     540

gctgctgaga tcttgtcgca gctgacttcc atgggcaaga gtgcagatca cgtgatggtt     600

ccagtcggcg gtggcggact tcttgcaggt gtggtcagct acatggctga tatggcacct     660

cgcactgcga tcgttggtat cgaaccagcg ggagcagcat ccatgcaggc tgcattgcac     720

aatggtggac caatcacttt ggagactgtt gatccctttg tggacggcgc agcagtcaaa     780

cgtgtcggag atctcaacta caccatcgtg gagaagaacc agggtcgcgt gcacatgatg     840

agcgcgaccg agggcgctgt gtgtactgag atgctcgatc tttaccaaaa cgaaggcatc     900

atcgcggagc ctgctggcgc gctgtctatc gctgggttga aggaaatgtc ctttgcacct     960

ggttctgtcg tggtgtgcat catctctggt ggcaacaacg atgtgctgcg ttatgcggaa    1020

atcgctgagc gctccttggt gcaccgcggt ttgaagcact acttcttggt gaacttcccg    1080

caaaagcctg gtcagttgcg tcacttcctg gaagatatcc tgggaccgga tgatgacatc    1140

acgctgtttg agtacctcaa gcgcaacaac cgtgagaccg gtactgcgtt ggtgggtatt    1200

cacttgagtg aagcatcagg attggattct ttgctggaac gtatggagga atcggcaatt    1260

gattcccgtc gcctcgagcc gggcacccct gagtacgaat acttgaccta a             1311

<210>    25
<211>    922
<212>    PRT
<213>    aceE from Corynebacterium glutamicum ATCC13032

<400>    25
Met Ala Asp Gln Ala Lys Leu Gly Gly Lys Pro Ser Asp Asp Ser Asn
1               5                   10                  15

Phe Ala Met Ile Arg Asp Gly Val Ala Ser Tyr Leu Asn Asp Ser Asp
            20                  25                  30

Pro Glu Glu Thr Asn Glu Trp Met Asp Ser Leu Asp Gly Leu Leu Gln
        35                  40                  45

Glu Ser Ser Pro Glu Arg Ala Arg Tyr Leu Met Leu Arg Leu Leu Glu

50                  55                  60

Arg Ala Ser Ala Lys Arg Val Ser Leu Pro Pro Met Thr Ser Thr Asp
65                  70                  75                  80

Tyr Val Asn Thr Ile Pro Thr Ser Met Glu Pro Glu Phe Pro Gly Asp
                85                  90                  95

Glu Glu Met Glu Lys Arg Tyr Arg Arg Trp Ile Arg Trp Asn Ala Ala
            100                 105                 110

Ile Met Val His Arg Ala Gln Arg Pro Gly Ile Gly Val Gly Gly His
        115                 120                 125

Ile Ser Thr Tyr Ala Gly Ala Ala Pro Leu Tyr Glu Val Gly Phe Asn
    130                 135                 140

His Phe Phe Arg Gly Lys Asp His Pro Gly Gly Gly Asp Gln Ile Phe
145                 150                 155                 160

Phe Gln Gly His Ala Ser Pro Gly Met Tyr Ala Arg Ala Phe Met Glu
                165                 170                 175

Gly Arg Leu Ser Glu Asp Asp Leu Asp Gly Phe Arg Gln Glu Val Ser
            180                 185                 190

Arg Glu Gln Gly Gly Ile Pro Ser Tyr Pro His Pro His Gly Met Lys
        195                 200                 205

Asp Phe Trp Glu Phe Pro Thr Val Ser Met Gly Leu Gly Pro Met Asp
    210                 215                 220

Ala Ile Tyr Gln Ala Arg Phe Asn Arg Tyr Leu Glu Asn Arg Gly Ile
225                 230                 235                 240

Lys Asp Thr Ser Asp Gln His Val Trp Ala Phe Leu Gly Asp Gly Glu
                245                 250                 255

Met Asp Glu Pro Glu Ser Arg Gly Leu Ile Gln Gln Ala Ala Leu Asn
            260                 265                 270

Asn Leu Asp Asn Leu Thr Phe Val Val Asn Cys Asn Leu Gln Arg Leu
        275                 280                 285

Asp Gly Pro Val Arg Gly Asn Thr Lys Ile Ile Gln Glu Leu Glu Ser
    290                 295                 300

Phe Phe Arg Gly Ala Gly Trp Ser Val Ile Lys Val Val Trp Gly Arg
305                 310                 315                 320

Glu Trp Asp Glu Leu Leu Glu Lys Asp Gln Asp Gly Ala Leu Val Glu
                325                 330                 335

Ile Met Asn Asn Thr Ser Asp Gly Asp Tyr Gln Thr Phe Lys Ala Asn
            340                 345                 350

Asp Gly Ala Tyr Val Arg Glu His Phe Phe Gly Arg Asp Pro Arg Thr
        355                 360                 365

Ala Lys Leu Val Glu Asn Met Thr Asp Glu Glu Ile Trp Lys Leu Pro
    370                 375                 380

Arg Gly Gly His Asp Tyr Arg Lys Val Tyr Ala Ala Tyr Lys Arg Ala

385                 390                 395                 400

Leu Glu Thr Lys Asp Arg Pro Thr Val Ile Leu Ala His Thr Ile Lys
                405                 410                 415

Gly Tyr Gly Leu Gly His Asn Phe Glu Gly Arg Asn Ala Thr His Gln
            420                 425                 430

Met Lys Lys Leu Thr Leu Asp Asp Leu Lys Leu Phe Arg Asp Lys Gln
        435                 440                 445

Gly Ile Pro Ile Thr Asp Glu Gln Leu Glu Lys Asp Pro Tyr Leu Pro
    450                 455                 460

Pro Tyr Tyr His Pro Gly Glu Asp Ala Pro Glu Ile Lys Tyr Met Lys
465                 470                 475                 480

Glu Arg Arg Ala Ala Leu Gly Gly Tyr Leu Pro Glu Arg Arg Glu Asn
                485                 490                 495

Tyr Asp Pro Ile Gln Val Pro Pro Leu Asp Lys Leu Arg Ser Val Arg
            500                 505                 510

Lys Gly Ser Gly Lys Gln Gln Ile Ala Thr Thr Met Ala Thr Val Arg
        515                 520                 525

Thr Phe Lys Glu Leu Met Arg Asp Lys Gly Leu Ala Asp Arg Leu Val
    530                 535                 540

Pro Ile Ile Pro Asp Glu Ala Arg Thr Phe Gly Leu Asp Ser Trp Phe
545                 550                 555                 560

Pro Thr Leu Lys Ile Tyr Asn Pro His Gly Gln Asn Tyr Val Pro Val
                565                 570                 575

Asp His Asp Leu Met Leu Ser Tyr Arg Glu Ala Pro Glu Gly Gln Ile
            580                 585                 590

Leu His Glu Gly Ile Asn Glu Ala Gly Ser Val Ala Ser Phe Ile Ala
        595                 600                 605

Ala Gly Thr Ser Tyr Ala Thr His Gly Lys Ala Met Ile Pro Leu Tyr
    610                 615                 620

Ile Phe Tyr Ser Met Phe Gly Phe Gln Arg Thr Gly Asp Ser Ile Trp
625                 630                 635                 640

Ala Ala Ala Asp Gln Met Ala Arg Gly Phe Leu Leu Gly Ala Thr Ala
                645                 650                 655

Gly Arg Thr Thr Leu Thr Gly Glu Gly Leu Gln His Met Asp Gly His
            660                 665                 670

Ser Pro Val Leu Ala Ser Thr Asn Glu Gly Val Glu Thr Tyr Asp Pro
        675                 680                 685

Ser Phe Ala Tyr Glu Ile Ala His Leu Val His Arg Gly Ile Asp Arg
    690                 695                 700

Met Tyr Gly Pro Gly Lys Gly Glu Asp Val Ile Tyr Tyr Ile Thr Ile
705                 710                 715                 720

Tyr Asn Glu Pro Thr Pro Gln Pro Ala Glu Pro Glu Gly Leu Asp Val

725                 730                 735

Glu Gly Leu His Lys Gly Ile Tyr Leu Tyr Ser Arg Gly Glu Gly Thr
            740                 745                 750

Gly His Glu Ala Asn Ile Leu Ala Ser Gly Val Gly Met Gln Trp Ala
        755                 760                 765

Leu Lys Ala Ala Ser Ile Leu Glu Ala Asp Tyr Gly Val Arg Ala Asn
    770                 775                 780

Ile Tyr Ser Ala Thr Ser Trp Val Asn Leu Ala Arg Asp Gly Ala Ala
785                 790                 795                 800

Arg Asn Lys Ala Gln Leu Arg Asn Pro Gly Ala Asp Ala Gly Glu Ala
                805                 810                 815

Phe Val Thr Thr Gln Leu Lys Gln Thr Ser Gly Pro Tyr Val Ala Val
            820                 825                 830

Ser Asp Phe Ser Thr Asp Leu Pro Asn Gln Ile Arg Glu Trp Val Pro
        835                 840                 845

Gly Asp Tyr Thr Val Leu Gly Ala Asp Gly Phe Gly Phe Ser Asp Thr
    850                 855                 860

Arg Pro Ala Ala Arg Arg Phe Phe Asn Ile Asp Ala Glu Ser Ile Val
865                 870                 875                 880

Val Ala Val Leu Asn Ser Leu Ala Arg Glu Gly Lys Ile Asp Val Ser
                885                 890                 895

Val Ala Ala Gln Ala Ala Glu Lys Phe Lys Leu Asp Asp Pro Thr Ser
            900                 905                 910

Val Ser Val Asp Pro Asn Ala Pro Glu Glu
        915                 920

<210>   26
<211>   2769
<212>   DNA
<213>   aceE from Corynebacterium glutamicum ATCC13032

<400>   26
atggccgatc aagcaaaact tggtggcaag ccctcggatg actctaactt cgcgatgatc      60

cgcgatggcg tggcatctta tttgaacgac tcagatccgg aggagaccaa cgagtggatg     120

gattcactcg acggattact ccaggagtct tctccagaac gtgctcgtta cctcatgctt     180

cgtttgcttg agcgtgcatc tgcaaagcgc gtatctcttc ccccaatgac gtcaaccgac      240

tacgtcaaca ccattccaac ctctatggaa cctgaattcc caggcgatga ggaaatggag     300

aagcgttacc gtcgttggat tcgctggaac gcagccatca tggttcaccg cgctcagcga     360

ccaggcatcg gcgtcggcgg acacatttcc acttacgcag gcgcagcccc tctgtacgaa     420

gttggcttca accacttctt ccgcggcaag gatcacccag gcggcggcga ccagatcttc     480

ttccagggcc acgcatcacc aggtatgtac gcacgtgcat tcatggaggg tcgcctttct     540

gaagacgatc tcgatggctt ccgtcaggaa gtttcccgtg agcagggtgg cattccgtcc 600

taccctcacc cacacggtat gaaggacttc tgggagttcc caactgtgtc catgggtctt 660

ggcccaatgg atgccattta ccaggcacgt ttcaaccgct acctcgaaaa ccgtggcatc 720

aaggacacct ctgaccagca cgtctgggcc ttccttggcg acggcgaaat ggacgagcca 780

gaatcacgtg gtctcatcca gcaggctgca ctgaacaacc tggacaacct gaccttcgtg 840

gttaactgca acctgcagcg tctcgacgga cctgtccgcg gtaacaccaa gatcatccag 900

gaactcgagt ccttcttccg tggcgcaggc tggtctgtga tcaaggttgt ttggggtcgc 960

gagtgggatg aacttctgga gaaggaccag gatggtgcac ttgttgagat catgaacaac 1020

acctccgatg gtgactacca gaccttcaag gctaacgacg gcgcatatgt tcgtgagcac 1080

ttcttcggac gtgacccacg caccgcaaag ctcgttgaga acatgaccga cgaagaaatc 1140

tggaagcttc cacgtggcgg ccacgattac cgcaaggttt acgcagccta caagcgagct 1200

cttgagacca aggatcgccc aaccgtcatc cttgctcaca ccattaaggg ctacggactc 1260

ggccacaact tcgaaggccg taacgcaacc caccagatga agaagctgac gcttgatgat 1320

ctgaagttgt tccgcgacaa gcagggcatc ccaatcaccg atgagcagct ggagaaggat 1380

ccttaccttc ctccttacta ccacccaggt gaagacgctc ctgaaatcaa gtacatgaag 1440

gaacgtcgcg cagcgctcgg tggctacctg ccagagcgtc gtgagaacta cgatccaatt 1500

caggttccac cactggataa gcttcgctct gtccgtaagg gctccggcaa gcagcagatc 1560

gctaccacca tggcgactgt tcgtaccttc aaggaactga tgcgcgataa gggcttggct 1620

gatcgccttg tcccaatcat tcctgatgag gcacgtacct tcggtcttga ctcttggttc 1680

ccaaccttga agatctacaa cccgcacggt cagaactacg tgcctgttga ccacgacctg 1740

atgctctcct accgtgaggc acctgaagga cagatcctgc acgaaggcat caacgaggct 1800

ggttccgtgg catcgttcat cgctgcgggt acctcctacg ccacccacgg caaggccatg 1860

attccgctgt acatcttcta ctcgatgttc ggattccagc gcaccggtga ctccatctgg 1920

gcagcagccg atcagatggc acgtggcttc ctcttgggcg ctaccgcagg tcgcaccacc 1980

ctgaccggtg aaggcctcca gcacatggat ggacactccc ctgtcttggc ttccaccaac 2040

gagggtgtcg agacctacga cccatccttt gcgtacgaga tcgcacacct ggttcaccgt 2100

ggcatcgacc gcatgtacgg cccaggcaag ggtgaagatg ttatctacta catcaccatc 2160

tacaacgagc caaccccaca gccagctgag ccagaaggac tggacgtaga aggcctgcac 2220

aagggcatct acctctactc ccgcggtgaa ggcaccggcc atgaggcaaa catcttggct 2280

tccggtgttg gtatgcagtg ggctctcaag gctgcatcca tccttgaggc tgactacgga 2340

gttcgtgcca acatttactc cgctacttct tgggttaact tggctcgcga tggcgctgct 2400

cgtaacaagg cacagctgcg caacccaggt gcagatgctg gcgaggcatt cgtaaccacc 2460

cagctgaagc agacctccgg cccatacgtt gcagtgtctg acttctccac tgatctgcca 2520

aaccagatcc gtgaatgggt cccaggcgac tacaccgttc tcggtgcaga tggcttcggt 2580

ttctctgata cccgcccagc tgctcgtcgc ttcttcaaca tcgacgctga gtccattgtt 2640

gttgcagtgc tgaactccct ggcacgcgaa ggcaagatcg acgtctccgt tgctgctcag 2700

gctgctgaga agttcaagtt ggatgatcct acgagtgttt ccgtagatcc aaacgctcct 2760

gaggaataa 2769

<210> 27
<211> 28
<212> DNA
<213> Artificial Sequence

<220>
<223> Pcj7-5-KpnI

<400> 27
gatggtacca ccccagaaac atcccagc 28

<210> 28
<211> 30
<212> DNA
<213> Artificial Sequence

<220>
<223> Pcj7-3 NdeI

<400> 28
cgatcatatg gagtgtttcc tttcgttggg 30

<210> 29
<211> 21
<212> DNA
<213> Artificial Sequence

<220>
<223> 117-F

<400> 29
ccacagccga caggatggtg a 21

<210> 30
<211> 21
<212> DNA
<213> Artificial Sequence

<220>
<223> 117-R

<400> 30

ctcagggtgt agcggttcgg t                                                21

<210>    31
<211>    34
<212>    DNA
<213>    leuB-5-cimA

<400>    31
ttgttgaatt aatctagagg tgacacccca gtgg                                    34

<210>    32
<211>    34
<212>    DNA
<213>    Artificial Sequence

<220>
<223>    leuB-3-leuC

<400>    32
tcgcagctgc caccgatatt tagctttgca gcgc                                    34

<210>    33
<211>    34
<212>    DNA
<213>    Artificial Sequence

<220>
<223>    leuC-5-leuB

<400>    33
gcgctgcaaa gctaaatatc ggtggcagct gcga                                    34

<210>    34
<211>    34
<212>    DNA
<213>    Artificial Sequence

<220>
<223>    leuD-3-XbaI

<400>    34
tggcggccgc tctagagctt tcgctatcag actg                                    34

<210>    35
<211>    25
<212>    DNA
<213>    Artificial Sequence

<220>
<223>    cimA-5-NdeI

<400>    35
gcatcatatg atggtaagga tattc                                              25

<210>	36
<211>	27
<212>	DNA
<213>	Artificial Sequence

<220>
<223>	cimA-3-XbaI


<400>	36
cgatctagat taattcaaca acatgtt	27


<210>	37
<211>	27
<212>	DNA
<213>	Artificial Sequence

<220>
<223>	ilvA-5-XbaI


<400>	37
gcatctagag aacacggaca atgccac	27


<210>	38
<211>	32
<212>	DNA
<213>	Artificial Sequence

<220>
<223>	ilvA-Del-R


<400>	38
caaacagcgt gatgtcctga gtgagctgcg ct	32


<210>	39
<211>	32
<212>	DNA
<213>	Artificial Sequence

<220>
<223>	ilvA-Del-F


<400>	39
agcgcagctc actcaggaca tcacgctgtt tg	32


<210>	40
<211>	29
<212>	DNA
<213>	Artificial Sequence

<220>
<223>	ilvA-3-XbaI


<400>	40

gcatctagaa cctgcggcac accttgggc                                    29

<210>    41
<211>    19
<212>    DNA
<213>    Artificial Sequence

<220>
<223>    Topo-F

<400>    41
gcagtgagcg caacgcaat                                              19

<210>    42
<211>    19
<212>    DNA
<213>    Artificial Sequence

<220>
<223>    Topo-R

<400>    42
cgttgtaaaa cgacggcca                                              19

<210>    43
<211>    19
<212>    DNA
<213>    Artificial Sequence

<220>
<223>    CFilvA

<400>    43
gatctgtgat gaggtgatg                                              19

<210>    44
<211>    19
<212>    DNA
<213>    Artificial Sequence

<220>
<223>    CRilvA

<400>    44
cgtccttcca tgactctca                                              19

<210>    45
<211>    26
<212>    DNA
<213>    Artificial Sequence

<220>
<223>    lysC-5-KpnI

<400>    45
tgaggtaccc atgcgattgt taatgc                                          26


<210>    46
<211>    28
<212>    DNA
<213>    Artificial Sequence

<220>
<223>    asd-3-SfoI


<400>    46
ctaggcgcca gtgtagcact caagcgga                                        28


<210>    47
<211>    27
<212>    DNA
<213>    Artificial Sequence

<220>
<223>    hom-5-BamHI


<400>    47
ctaggatccc tcaccattct caatggt                                         27


<210>    48
<211>    25
<212>    DNA
<213>    Artificial Sequence

<220>
<223>    thrB-3-BamHI


<400>    48
ctaggatccg ccttccttgt tgggc                                           25


<210>    49
<211>    27
<212>    DNA
<213>    Artificial Sequence

<220>
<223>    thrC-5-SpeI


<400>    49
tgcactagtg agaacataca ggttcca                                         27


<210>    50
<211>    32
<212>    DNA
<213>    Artificial Sequence

<220>

<223>    thrC-3-ilvA

<400>    50
ggcattgtcc gtgttcttac ttcacggaag tg                                    32

<210>    51
<211>    32
<212>    DNA
<213>    Artificial Sequence

<220>
<223>    ilvA-5-thrC

<400>    51
cacttccgtg aagtaagaac acggacaatg cc                                    32

<210>    52
<211>    29
<212>    DNA
<213>    Artificial Sequence

<220>
<223>    ilvA-3-SpeI

<400>    52
tgcactagta cctgcggcac accttgggc                                        29

## Claims

1. A method for producing L-isoleucine, comprising:
culturing a microorganism of the genus *Corynebacterium* comprising a protein having an activity of citramalate synthase in a medium; and
recovering L-isoleucine from the microorganism or medium.

2. The method of claim 1, wherein the citramalate synthase is derived from a microorganism of the genus *Methanocaldococcus.*

3. The method of claim 1, wherein the citramalate synthase has an amino acid sequence selected from the group consisting of SEQ ID NOS: 1, 3, 5, 7, 9, 11, 13, and 15.

4. The method of claim 1, wherein the microorganism of the genus *Corynebacterium* further comprises enhanced activities of 3-isopropylmalate dehydrogenase and 3-isopropylmalate dehydratase.

5. The method of claim 1, wherein the microorganism of the genus *Corynebacterium* further comprises inactivated pyruvate dehydrogenase.

6. The method of claim 1, wherein acetate is further provided in culturing the microorganism of the genus *Corynebacterium.*

7. The method of claim 1, wherein the microorganism of the genus *Corynebacterium* is *Corynebacterium glutamicum.*

8. A microorganism of the genus *Corynebacterium* having L-isoleucine producing ability, wherein the microorganism comprises a protein having an activity of citramalate synthase.

9. The microorganism of claim 8, wherein the citramalate synthase has an amino acid sequence selected from the group consisting of SEQ ID NOS: 1, 3, 5, 7, 9, 11, 13, and 15.

10. The microorganism of claim 8, wherein the microorganism further comprises enhanced activities of 3-isopropylmalate dehydrogenase and 3-isopropylmalate dehydratase.

11. The microorganism of claim 8, wherein the microorganism further comprises inactivated pyruvate dehydrogenase.

12. The microorganism of claim 8, wherein the microorganism is *Corynebacterium glutamicum.*
